# EUROPEAN PATENT APPLICATION

(11) **EP 1 415 997 A1**
(43) Date of publication of application: **06.05.2004**
(21) Application number: 02753198.7
(22) Date of filing: 18.07.2002
(51) Int. Cl.: C07K 14/47, A61P 43/00, C12N 15/12, C12N 1/19, C12N 1/21, C12N 5/10, C12P 21/02, C12Q 1/68, C07K 16/18, A61K 48/00, A61K 38/00, A61K 45/00, A61P 3/10, A61P 25/28, A61P 35/00, A61P 1/02

(54) **POLYPEPTIDES RELATING TO SIGNAL TRANSFER OF ADVANCED GLYCATION END PRODUCT RECEPTOR**

(30) Priority: 19.07.2001 JP 2001219122
(71) Applicant: Mitsubishi Pharma Corporation, Osaka-shi, Osaka 541-0046 (JP)
(72) Inventor: TSUCHIDA, Jun-ichi, MITSUBISHI PHARMA CORPORATION, Tokyo 103-8405 (JP)
(74) Representative: von Kreisler, Alek, Dipl.-Chem.
(86) International application number: PCT/JP2002/007344
(87) International publication number: WO 2003/008446

(57) **Abstract**

It is intended to provide polypeptides which directly or indirectly bind to a cytoplasmic domain of RAGE and inhibits the signal transduction from binding of a ligand to RAGE (the receptor for advanced glycation endproduct) through activation of NFκB. Typical examples thereof are polypeptides respectively having amino acid sequences represented by SEQ ID NOS: 1 to 32, SEQ ID NOS:67 to 79 and SEQ ID NOS: 80 to 86 in Sequence Listing or the amino acid sequence including deletion, substitution or addition of one to several amino acids. Also, polypeptides involved in the signal transduction of RAGE are provided. Moreover, a pharmaceutical for gene therapy using polynucleotides encoding these polypeptides: a method for screening a useful compound using these polypeptides: and pharmaceutical composition and diagnostic reagent are also provided.

## Description

### Technical Field

The present invention relates to novel polypeptides. In particular, the present invention relates to novel polypeptides involved in a signal transduction of the receptor for an advanced glycation end product (AGE) (hereinafter, the receptor is also referred to as "RAGE") and a polynucleotide encoding the same.

### Background Art

A non-enzymatic reaction of glucose or another reducing sugar with an amino group in a protein or in a lipid generates a Schiff base, and then an irreversible reaction thereof results in advanced glycation endproducts (AGE). AGE is produced and accumulated by aging or hyperglycemia, and it cause denaturation of proteins, production of active oxygen, and activation of cells and inflammation. The accumulation of AGE is suggested in various kinds of diseases including diabetes, Alzheimer's Disease, dialysis amyloidosis, and also in natural aging.

A binding to a specific receptor is regarded as one of the mechanisms of AGE's action. Several receptors for AGE are known. Among them, RAGE (the receptor for AGE: AGE receptor) is considered to be a main receptor. RAGE is a single transmembrane receptor cloned in 1992, having three immunoglobulin domains on the extracellular domain. Examples of ligands thereof include AGE, amphoterin, and β-amyloid.

It is known in the art that the binding of AGE to RAGE causes activation of NFκB by an oxidative stress. However, a signal transduction from RAGE through NFκB has not been revealed up to now.

If a molecule involved in such a pathway is identified, it may be possible to block a signal transduction of RAGE and thereby, inhibit the onset of a disease such as diabetes, a diabetic complication, Alzheimer's Disease, dialysis amyloidosis, a cancer, a periodontal disease, or aging, by (i) exogenously introducing the molecule, (ii) inhibiting the expression of the molecule by a small molecule, (iii) inhibiting the function of the molecule by a small molecule, or the like,

### Disclosure of the Invention

That is, an object of the present invention is to obtain a molecule involved in a signal transduction of RAGE and to use the molecule for treatment of diseases such as those listed above or for screening of pharmaceuticals. In particular, (i) a gene therapy in which the obtained gene is introduced into human body, (ii) screening or evaluation of pharmaceuticals in cells in which the obtained gene is introduced, or the like can be exemplified.

For finding out a protein to interact with a cytoplasmic domain of RAGE, the inventors of the present invention have screened human kidney cDNA libraries using a yeast two-hybrid system. Consequently, 31 polynucleotides predicted to bind to a cytoplasmic domain of RAGE have been obtained. Furthermore, seven polynucleotides have been obtained as a result of screening human brain cDNA libraries, and thus the present invention has been completed.

That is, a gist of the present invention resides in a polypeptide, which is a substantially purified polypeptide, and is characterized by:
(a) directly or indirectly binds to a cytoplasmic domain of receptor for an advanced glycation end product (AGE); and
(b) inhibits a signal transduction frombinding of a ligand to receptor for the advanced glycation endproduct (AGE) through activation of NFκB.

According to a preferable embodiment of the above, there can be mentioned an amino acid sequence selected from SEQ ID NOS: 1 to 32, SEQ ID NOS: 67 to 79, and SEQ ID NOS: 80 to 86 in the Sequence Listing or the amino acid sequence including deletion, substitution, or addition of one or several amino acids. According to a further preferable embodiment of the present invention, there can be mentioned an amino acid sequence selected from SEQ ID NOS: 11, 12, 29, and 30 in the Sequence Listing or the amino acid sequence including deletion, substitution, or addition of one or several amino acids.

As a second gist of the present invention, a polynucleotide which encodes the above-described polypeptide can be mentioned. According to a preferable embodiment thereof, there can be mentioned a nucleotide sequence represented by one of SEQ ID NOS: 33 to 63 and SEQ ID NOS: 87 to 93 or a nucleotide sequence hybridizable with the polynucleotide represented by the nucleotide sequence under a stringent condition. According to a further preferable embodiment, there can be mentioned a nucleotide sequence represented by one of SEQ ID NOS: 43, 44, 61, and 62 or a nucleotide sequence hybridizable with the polynucleotide represented by the nucleotide sequence under a stringent condition.

Further, as a third gist of the present invention, there can be mentioned a pharmaceutical for a gene therapy, comprising a vector which is expressible in an animal and contains the above-described polynucleotide. According to a preferable embodiment thereof, there can be mentioned a pharmaceutical for treating diabetes, a diabetic complication, Alzheimer's Disease, dialysis amyloidosis, cancer, periodontal disease, or an aging-related disease.

Further, as another gist of the present invention, there can be mentioned a vector containing the above-mentioned polynucleotide; a microorganism or cell which is transformed with the vector; and a method for producing the polypeptide, comprising culturing the microorganism or cell and isolating the polypeptide from the culture.

As a fourth gist of the present invention, there can be mentioned a method for screening a substance that inhibits or accelerates the biding of the above-mentioned polypeptide to a cytoplasmic domain of the receptor for AGE, comprising placing a target of screening in a screening system that contains a cytoplasmic domain of the receptor for AGE and the polypeptide and measuring a degree of inhibition or acceleration of the binding of the polypeptide to the cytoplasmic domain of the receptor for AGE.

As a screening method, there can be mentioned as another gist of the present invention, a method of screening a substance that enhances or inhibits a function of the polypeptide or a substance.that increases or decreases an amount of the polypeptide, comprising placing a target of screening in a screening system that contains the above-mentioned polypeptides and measuring a degree of enhancement or inhibition of the function of the polypeptide or a degree of increase or decrease in the amount of the polypeptide.

As a fifth gist of the present invention, there can be mentioned a compound which is obtainable by the screening method described above, and according to a preferable embodiment thereof, there can be mentioned a compound characterized by:
(a) directly or indirectly binds to a cytoplasmic domain of the receptor for an advanced glycation endproduct (AGE); and
(b) inhibits a signal transduction frombinding of a ligand with the receptor for advanced glycation endproduct (AGE) through activation of NFκB.

Further, as another gist of the present invention, there can be mentioned a pharmaceutical composition comprising, as an effective ingredient, the substance selected from the group consisting of the above-mentioned compound, a salt thereof, a hydrate thereof, and a solvate thereof. According to a preferable embodiment, the pharmaceutical composition may be used for treating a disease selected from diabetes, a diabetic complication, Alzheimer's Disease, dialysis amyloidosis, a cancer, a periodontal disease, and an aging-related disease.

Further, as another gist of the present invention, there can be mentioned an antibody which can specifically bind to the polypeptide; a polynucleotide which is represented by a nucleotide sequence having at least 20 sequential nucleotides in any of the sequences of SEQ ID NOS: 33 to 63 and SEQ ID NOS: 87 to 93, preferably in any of the sequences of SEQ ID NOS: 43, 44, 61, and 62 in the Sequence Listing, a sequence complementary to the nucleotide sequence, and a nucleotide sequence hybridizable with the nucleotide sequence under a stringent condition; a probe comprising the polynucleotide and a label; and a diagnostic reagent including the probe. According to a preferable embodiment, the diagnostic reagent may be used for a diagnosis of diabetes, a diabetic complication, Alzheimer's Disease, dialysis amyloidosis, a cancer, a periodontal disease, and an aging-related disease.

### Brief Description of the Drawing

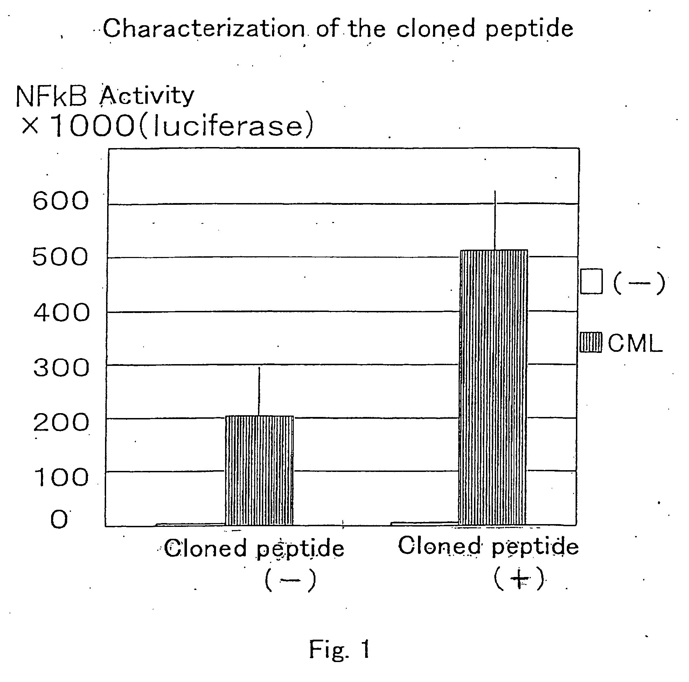

Fig. 1 is a diagram that illustrates functions of the cloned polypeptides.

### Best Mode for Carrying Out the Invention

Hereinafter, the present invention is described specifically. The polypeptide of the present invention is a substantially pure polypeptide which can directly or indirectly bind to a cytoplasmic domain of RAGE and inhibit signal transduction from binding of a ligand to RAGE through activation of NFκB.

The "direct biding" referred to herein means a direct binding of the polypeptide of the present invention to a cytoplasmic domain of RAGE, and the "indirect biding" means a binding of the polypeptide of the present invention to a cytoplasmic domain of RAGE through a protein or the like preexisting in an environment where the polypeptide of the present invention can bind to a cytoplasmic domain of RAGE (in an assay system or a human body, etc.). As "ligand", there can be mentioned AGE, amphoterin, β-amyloid, or the like. For instance, when the polypeptide of the present invention is used for a method of screening pharmaceuticals or for a gene therapy which will be described below, the ligand can be selected as appropriate depending on a target disease. For example, AGE can be selected when the target disease is diabetes or a diabetic complication, amphoterin can be selected when the target disease is a cancer, and β-amyloid can be selected when the target disease is Alzheimer's Disease.

In the present invention, the "polypeptides" includes those typically recognized as peptides, oligopeptides, polypeptides, and proteins in the art. Natural proteins and chemically-synthesized or recombinantly-engineered polypeptides and peptides are also included. The polypeptide may or may not be subjected to a post-translational modification such as glycosylation or phosphorylation.

A precursor of the polypeptide of the present invention is also included in the polypeptide of the present invention as far as it has the physiological activities as described above. Examples of such a precursor include one having one or more amino acids added to the N-terminus and (or) the C-terminus of the peptide of the present invention.

Furthermore, the polypeptide of the present invention may be bound to polyethylene glycol for prolonging the half-period thereof, or fused with a secretory sequence or a leader sequence for secretion. Alternatively, the polypeptide of the present invention may be produced as a fusion polypeptide for purification.

A fragment characterized by the structural or functional properties of the polypeptide of the present invention is also useful.

A physiologically acceptable acid-added salt of the polypeptide of the present invention or of a precursor of the polypeptide is also included in the present invention. As a acid-added salt, there can be mentioned a salt with an inorganic acid such as hydrochloric acid, phosphoric acid, or sulfuric acid; and a salt with an organic acid such as acetic acid, formic acid, fumaric acid, maleic acid, succinic acid, citric acid, tartaric acid, malic acid, benzoic acid, orbenzenesulfonic acid.

The "amino acid sequence including deletion, substitution or deletion of one or several amino acids " of the present invention means an amino acid sequence of a mutant of an allelic mutation or a mutation found in nature, or an artificial mutation or a mutation obtainable by using recombinant technology. All of the amino acid sequences to be included in the present invention are polypeptides having the same activity as that of the novel polypeptide molecule of the present invention. Even if only one amino acid residue is modified, an amino acid sequence including variation which causes the loss of the activity is not included in the present invention.

The "polynucleotide" of the present invention is a polypeptide that encodes the polypeptide of the present invention as described above. Specifically, the polynucleotide of the present invention is a polynucleotide encoding an amino acid sequence represented by one of SEQ ID NOS: 1 to 32, SEQ ID NOS: 67 to 79, and SEQ ID NOS: 80 to 86, preferably one of SEQ ID NOS: 11, 12, 29, and 30 in the Sequence Listing. Specifically, such a polynucleotide can be exemplified by a polynucleotide having a nucleotide sequence represented by one of SEQ ID NOS: 33 to 63 and SEQ ID NOS: 87 to 93, preferably one of SEQ ID NOS: 43, 44, 61, and 62. However, as far as the same amino acid sequence is encoded, each codon may be substituted with other equivalent codon.

The polynucleotide of the present invention may be a polynucleotide encoding a polypeptide having substantially the same amino acid sequence as the amino acid sequence represented by one of SEQ ID NOS: 1 to 32, SEQ ID NOS: 67 to 79, and SEQ ID NOS: 80 to 86, preferably one of SEQ ID NOS: 11, 12, 29, and 30. Such a polynucleotide is preferably a polynucleotide having at least 80% homology to the nucleotide sequence represented by one of SEQ ID NOS: 33 to 63 and SEQ ID NOS: 87 to 93, preferably one of SEQ ID NOS: 43, 44, 61, and 62, or its complementary nucleotide sequence. A polynucleotide having 90% homology is more preferable, and in particular, the most preferable is a polynucleotide having 95% or more homology. As a polynucleotide having 95% or more, preferably 97% or more homology, there can be mentioned a polynucleotide hybridizable with the polynucleotide represented by one of SEQ ID NOS: 33 to 63 and SEQ ID NOS: 87 to 93, preferably one of SEQ ID NOS: 43, 44, 61, and 62 or a probe prepared from the sequence under a stringent condition.

Furthermore, the present invention includes a polynucleotide that encodes a fragment characterized by the structural or functional properties of the polypeptide of the present invention. Such a polynucleotide fragment and a polynucleotide hybridizable with a polynucleotide that encodes the fragment are useful as PCR primers as well as probes for detecting a DNA that encodes the peptide of the present invention.

Hereinafter, the characteristic features included in the present invention are described with reference to Examples described below as a representative example. However, the present invention is not limited to these examples.

For the purpose of obtaining a novel molecule involved in the signal transduction from binding of AGE to RAGE through activation of NFκB, a gene for a cytoplasmic domain of RAGE was amplified by a PCR method, as shown in Example 1 described later. Then, using the gene for a cytoplasmic domain of RAGE, human kidney cDNA libraries and brain cDNA libraries were screened by a yeast two-hybrid method using the LexA system. Consequently, so far as the inventor of the present invention knows, a polynucleotide was obtained, which encodes a novel polypeptide having no homology to any protein that has been reported so far.

In this way, 31 polynucleotides and 7 polynucleotides predicted to bind to a cytoplasmic domain of RAGE were obtained. Their nucleotide sequences are represented by one of SEQ ID NOS: 33 to 63 and 87 to 93, preferably one of SEQ ID NOS: 43, 44, 61, and 62 in the Sequence Listing.

The nucleotide sequence of the polynucleotide cloned as described above can be linked with a vector which is expressible in a host cell and contains transcriptional control activities such as a promoter, an operator, or an enhancer; a termination sequence; and other regulatory sequences for controlling the expression of RAGE.

A recombinant vector containing the polynucleotide of the present invention is used for the production of the polypeptide of the present invention. The vector of the present invention to be used is one suitable for retention, amplification, and expression of a polynucleotide in a host cell. The cloned polynucleotide that encodes the polypeptide of the present invention can be inserted into a vector directly or after being digested with a restriction enzyme or after being linked to a linker. A DNA has a translation initiation codon (ATG) on its 5'-terminal side and a translation termination codon (TAA, TGA, or TAG) on its 3'-terminal side. A DNA is located downstream from a promoter in the expression vector.

Examples of such a vector include: plasmids derived from *Escherichia coli* (such as pBR322, pBR325, and pUC12), plasmids derived from *Bacillus subtilis* (such as pUB110 and pC194), plasmids derived from the genus *Streptomyces,* and plasmids derived from the genus *Salmonella*; plasmids derived from yeast episomes and host chromosome elements (such as YCp plasmids and pYAC plasmids), bacteriophages such as λ-phage and vectors derived from viruses such as Vaccinia virus, Adenovirus, Retrovirus, and Baculovirus. Many of those vectors are commercially available.

A DNA sequence in the recombinant vector is operably-linked to an appropriate expression control sequence (promoter). Examples of such a promoter include: phage λPL promoter and T7 promoter; *Escherichia coli* lac, trp, lpp, and c promoters; *Bacillus subtilis* SPO1 promoter and penP promoter; yeast PHO5 promoter, PGK promoter, GAP promoter, ADH1 promoter, SUC2 promoter, GAL4 promoter, and MFα promoter; insect cell polyhedron promoter and P10 promoter; and animal cell SV40 early or late promoter, retrovirus LTR promoter, CMV promoter, HSV-TK promoter, and metallothionein promoter.

In general, the expression vector contains an expression control region to be regulated by a repressor-binding region, an enhancer, or the like. In addition, the expression vector contains a selection marker. Examples of appropriate markers include a dihydrofolate reductase (dhfr) gene and a neomycin-resistance gene for eukaryotic cells, and a tetracycline or ampicillin resistance gene for bacterial cells. The dhfr gene provides a transformed cell with methotrexate resistance, while the neomycin-resistance gene provides a transformed cell with G418 resistance. When a host cell is a dhfr gene-deficient CHO cell and the dhfr gene is used as a selection marker, a transformant can be selected in a thymidine-free medium. In this case, a resistant strain can be selected by culturing cells under a gradually increasing concentration of methotrexate (MTX). Thus, a DNA that encodes the peptide of the present invention is amplified in the cell simultaneously with the amplification of the dhfr gene, thereby a CHO (dhfr -) cell with high level expression can be obtained.

The recombinant vector of the present invention is constructed in a way that a signal sequence is added to the N terminus of the peptide, if required. Such a signal sequence may be: a PhoA or OmpA signal sequence or the like in the case of an *Escherichia coli* host; an Mfα or SUC2 signal sequence or the like in the case of a yeast host; and an α-interferon signal sequence or the like in the case of an animal host.

The present invention also relates to a host cell that harbors the recombinant vector as described above. Examples of the host cells include mammalian cells, plant cells, insect cells, yeast cells, eukaryotic cells such as *Aspergius* fungi, and prokaryotic cells such bacterial cells. The recombinant vector of the present invention can be introduced into the host cell by means of calcium-phosphate transfection, electroporation, transduction, infection, or the like.

Under the control of the promoter as described above, the peptide of the present invention can be expressed in hosts such as mammalian cells, yeasts, and bacteria as described above.

Examples of a prokaryotic host include *Escherichia coli, Bacillus subtilis, Salmonella, Pseudomonas, Streptomyces,* and *Staphylococcus*. Examples of the yeasts include *Saccharomyces cerevisiae, Schizosaccharomyces pombe,* and *Pichia pastoris.*

The transformed prokaryotic host is proliferated, and in a case of using a vector containing an inducible promoter, a temperature or a chemical inducer can initiate the induction. Then, the cells are cultured in a liquid medium containing a carbon source (such as glucose, dextran, or soluble starch), a nitrogen source (such as ammonium salt, nitrate salt, peptone, casein, meat extract, or bean cake), and an inorganic substance (such as calcium chloride, sodium dihydrogen phosphate, or magnesium chloride) at a suitable pH (i.e., at a pH of about 5 to 8) for a suitable period (i.e., for about 3 to 24 hours). The suitable culture temperature is about 14 to 43°C for *Escherichia coli* and about 30 to 40°C for bacteria of the genus *Bacillus.* The cell is physically or chemically disrupted after cultivation, and then the peptide of the present invention is purified from the resulting crude extract.

The transformed yeast is cultured in a medium such as a minimal medium at a pH of about 5 to 8 and at a temperature of about 20 to 35°C for about 24 to 72 hours.

Insect cells to be used are Sf cells, MG1 cells, or the like when the virus is AcNPV (Autographa californica NPV), larval silkworms, silkworm cultured cells (BM-N cells) or the like when the virus is Bombyx polynucleosis virus (BmPV). For the silkworm cells, a medium such as a TC-10 medium containing a heat-inactivated 10% fetal bovine serum is used, and the cells are cultured to be confluent at about 27°C, then the cells are subjected to passage.

Examples of the mammalian cell include COS-7 cell, mouse AtT-20 cell, rat GH3 cell, rat MtT cell, mouse MIN6 cell, Vero cell, C127 cell, CHO cell, dhfr-gene defect CHO cell, HeLa cell, L cell, BHK cell, BALB3T3 cell, 293 cell, Bowes melanoma cell and the like.

Expression vectors for mammalian cell include a replication origin, a promoter (such as the above-mentioned SV40 early or late promoter, retrovirus LTR promoter, CMV promoter, HSV-TK promoter, or metallothionein promoter), an enhancer (such as SV40 enhancer, adenovirus enhancer, or cytomegalovirus early promoter), a selection marker (such as the above-mentioned dhfr gene or neomycin-resistance gene), a libosome binding site, a polyadenylationsite(such asSV40 polyadenylationsite),splice donor and acceptor sites (such as a DNA sequence derived from SV40 splice site), a transcription termination sequence, and a 5'-non-transcription sequence.

As such a vector, a plasmid vector, a single- or double-stranded phage vector, a single- or double-stranded RNA or DNA virus vector, or the like can be used. As a medium for the transformed mammalian cells, an MEM medium, a DMEM medium, an RPMI-1640 medium, or the like, which contains a fetal bovine serum at a concentration of about 5 to 20%, can be used. The culture of the cells is performed at a pH of about 6 to 8 and a temperature of 30 to 40°C for about 15 to 72 hours.

When the mammalian cells are used as hosts, the polypeptide of the present invention can be collected and purified from a culture of the recombinant cells using ammonium sulfate or ethanol precipitation, acid extraction, anion- or cation-exchange chromatography, hydrophobic interaction chromatography, or the like.

The polypeptide of the present invention may not be glycosylated. In addition, depending on hosts, a polypeptide having methionine at its N-terminus can be obtained.

Furthermore, the polynucleotide of the present invention is useful in a gene therapy for treating a disease such as diabetes, a diabetic complication, Alzheimer's Disease, dialysis amyloidosis, a cancer, aperiodontaldisease, or an aging-related disease. The "gene therapy" means the administration of a gene or gene-incorporated cells into the human body for treating a disease. For a pharmaceutical use, any of a purified polynucleotide, a recombinant, a culture liquor of a transformant, an isolated transformant, a treated-transformant, a fixed-transformant, a crude enzyme solution, an enzyme-treated product, etc. may be used.

When the polynucleotide of the present invention is used for a gene therapy, various technologies conventionally used for a gene therapy can be employed. Specifically, a gene containing the polynucleotide of the present invention can be expressed in the body by implanting the expression vector for the polynucleotide of the present invention, which is obtained from a virus vector such as a retrovirus vector, an adenovirus vector, an AAV vector, or a herpes virus vector or which is obtained by a membrane-fusion liposome method, into bone marrow cells of a patient suffering from a disease such as diabetes, a diabetic complication, Alzheimer's Disease, dialysis amyloidosis, a cancer, a periodontal disease, or an aging-related disease (hereinafter, which may be collectively referred to as "the disease"), according to a method such as one described in JP 09-501046 A or a method based thereon; by administering the expression vector into the muscle tissue, blood system, intestine, lung, or the like of a patient suffering fromthe disease according to a method such as one described in JP 09-505084 A or a method based thereon; or by administering the expression vector into the cerebrospinal fluid of a patient suffering from the disease according to a method such as one described in JP 09-505561 A or a method based thereon. Furthermore, an offspring of the patient can be prevented from suffering from the disease by incorporating the gene containing the polynucleotide of the present invention into the egg cell of the patient suffering from the disease.

The polypeptide or polynucleotide of the present invention is useful for identifying a compound which can modulate control of the signal transduction from binding of a ligand to RAGE through activation of NFκB. That is, the present invention provides a method of screening a substance that inhibits or accelerates a binding of the polypeptide of the present invention to a cytoplasmic domain of RAGE. The method includes: placing a target of screening in a screening system containing a cytoplasmic domain of RAGE and the polypeptide of the present invention; andmeasuring the degree of inhibition or acceleration of the binding of the polypeptide of the present invention to a cytoplasmic domain of RAGE.

In the present invention, the "target of screening" is not particularly limited as far as it is a substance available for a screening, the target may be a high molecule as well as a low molecule. For instance, the target of screening may be a peptide, an analog thereof, a microbial culture liquor, or an organic compound. In the present invention, "screening" is used in the meaning of including an assay.

The screening system is not limited as far as it is a method typically used in the art, for instance, an experimental system such as a yeast two-hybrid system can be used. That is, the interaction between the polypeptide of the present invention and a cytoplasmic domain of RAGE is monitored by the yeast two-hybrid activity, and then a substance that inhibits or enhances the reporter activity thereof may be screed. In an alternative method, using the BIAcore (manufactured by BIACORE K. K. ) , a change in their interaction is monitored in the presence of a substance to be provided as a screening target to identify the influence of the substance on their interaction. In the screening described herein, not only a polypeptide having the full-length polypeptide of the present invention having an amino acid sequence selected from SEQ ID NOS: 1 to 32, SEQ ID NOS: 67 to 79, and SEQ ID NOS: 80 to 86 in the Sequence Listing but also a partial peptide having at least five amino acid residues can be used as far as the target substance can be screened in the above method. Preferably, it can be also possible to use not only a polypeptide having the full-length polypeptide of the present invention having an amino acid sequence selected from SEQ ID NOS: 11, 12, 29, and 30 but also a partial polypeptide having at least five residues as far as the target substance can be screened in the above method.

In the present invention, "measure the degree of inhibition of binding" is used in the meaning of including measuring the presence or absence of the binding.

The methods as described above are methods in which a cytoplasmic domain of RAGE is provided in the screening system. However, in the present invention, it is also possible to perform a method in which a cytoplasmic domain of RAGE is not provided in a screening system. That is, there is provided a method of screening a substance that enhances or inhibits the function of the polypeptide of the present invention or a substance that increases or decreases an amount of the polypeptide of the present invention, which method comprising: placing a target substance in a screening system containing the polypeptide of the present invention; and measuring the degree of enhancement or inhibition of the function of the polypeptide of the present invention or measuring the degree of an increase or decrease in an amount of the polypeptide of the present invention.

The screening target, the definition of the screening, and the screening system are as described above. "Measuring the degree of enhancement or inhibition of the function" and "measuring the degree of an increase or decrease in an amount" are used in the meaning of measuring the presence or absence of enhancement or inhibition of the function and measuring the presence or absence of an increase or decrease in the amount, respectively.

The screening system is not limited as far as it is a method conventionally used in the art, for instance, a vector containing the polypeptide of the present invention is introduced into a cell in which pNFk-luc (STRATAGENE Co., Ltd.) is introduced; and then, a substance that enhances or inhibits the NFκB activity is screened.

As described above, the polypeptide of the present invention is involved in the signal transduction from RAGE through NFκB, so that the screening method of the present invention can be used as a method of screening a substance useful as a preventive agent and/or a therapeutic agent for treating a disease such as diabetes, a diabetic complication, Alzheimer' s Disease, dialysis amyloidosis, a cancer, a periodontal disease, or an aging-related disease.

The compounds obtainable by using the screening method of the present invention and the salts thereof, and their hydrates and solvates are compounds that modify the binding of the polypeptide of the present invention to a cytoplasmic domain of RAGE (i.e., the compounds inhibit or accelerate the binding) or compounds having the cell-stimulating activities caused by the interaction between the polypeptide of the present invention and a cytoplasmic domain of RAGE. Examples of the compounds include peptides, proteins, fermented products, non-peptide compounds, and synthetic compounds. These compounds may be novel or known compounds.

As a pharmaceutical composition, for example, the compound itself may be administered alone. It is preferable to prepare a pharmaceutical composition containing the above compound as an effective ingredient by using a pharmaceutically acceptable pharmaceutical additive, and administrate the composition. The pharmaceutical composition may be orally or parenterally administered in a form of a pharmaceutical composition or preparation (for example, a tablet, a pill agent, a capsule, a granule, powder, syrup, an emulsion agent, an elixir agent, a suspending agent, a resolvent, an injection, a drop, or a suppository) which is obtainable by mixing the compound of the present invention with a pharmaceutically acceptable carrier (for example, an excipient, a binder, a disintegrating agent, a flavoring substance, a deodorizing substance, an emulsifier, an attenuant, or a solubilizer). The pharmaceutical composition can be manufactured according to the conventional method. In this specification, "parenterally" covers a hypodermic injection, an intravenous injection, an intramuscular injection, an intraperitoneal injection, and a drip method. A composition for injection can be prepared by the method known in the art. A rectal suppository can be produced by mixing the pharmaceutical compound with a suitable excipient. Examples of solid formulation for oral administration include those as described above such as powder, a granule, a tablet, a pill agent, and a capsule. Examples of liquid formulations for oral administration include an emulsion agent, syrup, an elixir agent, a suspending agent, and a solution agent, each of which is acceptable as a medicine.

Furthermore, each of the preparations as described above can be prepared by conventional procedures. The clinical dosage of the pharmaceutical of the present invention is determined by appropriately increasing or decreasing on the basis of the substance used as an effective ingredient, age, condition, symptom, the presence or absence of simultaneous administration, and so on. The above-described dosage per day may be administered once a day or two or several times per day with appropriate intervals. Alternatively, the dosage may be intermittently administered every several days.

On the basis of the nucleotide sequence information about the polynucleotide of the present invention, it is possible to obtain an antisence specific to a polynucleotide complementary to the sequence. The "antisence" referred to herein means a polynucleotide complementary to at least a part of mRNA or DNA that encodes the polynucleotide of the present invention, which inhibits the transcription and translation ofthe polynucleotide of the present invention. Furthermore, using the transformant of the present invention, the effects of the antisence can be also confirmed. For such an antisense, there can be used not only typical DNAs and RNAs but also any other sequence-specific nucleotide-like molecule considered to have antisence effects.

The polynucleotide and polypeptide of the present invention can be used for diagnosing the above-described diseases. An antibody specific to the polypeptide of the present invention can be obtained by using proteins which is produced in large amounts in a cell transformed by the polynucleotide of the present invention. As an immunizing antigen, the full-length polypeptide of the present invention or a peptide fragment consisting of at least five sequential amino acid residues of the amino acid sequence of the polypeptide of the present invention may be used. In the present invention, the antibodies include, in addition to the complete antibodies, all of the molecules which are produced from original antibody and considered to have antibody effects, for example, Fab, F(ab')2, Fv, and scFv fragments that contain the respective antigen-binding sites.

Using the antibodies as described above, it is possible to construct the system of ELISA or RIA or the system of western blotting for detecting the polypeptide of the present invention in cells and tissues. For instance, such a detecting system can be used for diagnosing the diseases as described in the explanation of the screening method.

Furthermore, in the present invention, the expression of the polypeptide of the present invention in cells or tissues can be detected using a probe that contains a suitable label and the polynucleotide represented by a nucleotide sequence comprising at least 20 sequential nucleotides in the sequence of any of SEQ ID NOS: 33 to 63 and SEQ ID NOS: 87 to 93, preferably any of SEQ ID NOS: 43, 44, 61, and 62 in the Sequence Listing, a complementary sequence thereof, and a nucleotide sequence hybridizable with the nucleotide sequence under a stringent condition. Therefore, such a probe can be also used in the assay for diagnosis. A label used in this case is not particularly limited as far as it can be used in the conventional assay for the diagnosis. As such a label, for example, avidin or biotin, an enzyme such as peroxidase, a radioactive isotope, a fluorescent substance, or an antigen can be used. Using those labels, the nucleotide sequences are labeled by a method conventionally used in the art, and the labels can be detected by suitable methods, respectively.

### Examples

Hereinafter, the present invention will be described in more detail with reference to the examples. However, the present invention is not limited only to these examples.

### Example 1

### Cloning of a protein which interacts with a cytoplasmic domain of RAGE by yeast two-hybrid method

A gene for a cytoplasmic domain of RAGE (SEQ ID NO: 64 in the Sequence Listing) (J. Biol. Chem 1992 Jul 25, 267 (21): 14998-5004) was amplified using synthetic oligonucleotides: 5'-ACGTGAATTCAGGCGGCAACGCCGAGGAG-3' (SEQ ID NO: 65 in the Sequence Listing) and 5'-CGATCTCGAGTCAAGGCCCTCCAGTACTACTC-3' (SEQ ID NO: 66 in the Sequence Listing), and was then digested with restriction enzymes EcoRI and XhoI. Also, a vector pHybLex / Zeo (manufactured by Invitrogen Co., Ltd.) was digested with therestriction enzymes EcoRI and XhoI. Subsequently, the both genes were subjected to a 14-hour ligation reaction using Takara Ligation Kit ver. 2 (manufactured by Takara Shuzo Co., Ltd.) and used for transforming *E*.*coli* strain DH5α (manufactured by Takara Shuzo Co., Ltd.), thereby colonies were obtained. Consequently, a vector (pHybLex / Zeo-RAGECD) in which the cytoplasmic domain of RAGE is incorporated into the pHybLex /Zeowas obtained. Using a DNA sequencer (manuf actured by Beckman Co.; Ltd.), the sequence of the cytoplasmic domain of RAGE was confirmed.

The vector pHybLex / Zeo-RAGECD obtained as described above was introduced into Yeast L40 strain attached to the MATCH MAKER kit which is described below, and then, a clone into which the pHybLex / Zeo-RAGECD had been introduced was selected.

Then, the Human kidney MATCHMAKER LexA library (manufactured by Clontech Co., Ltd.) was introduced to the above-described strain and 10⁶ colonies were screened. At the first screening, a histidine-deficient mediumwas used and about 200 colonies were selected based on the expression of a histidine gene, which was a reporter of the interaction. At the second screening, 31 colonies were obtained based on the expression of LacZ, which was another reporter of the interaction. Finally, 31 clones each of which had been confirmed to surely have two kinds of reporter activities were selected. The above procedures were carried out on the basis of protocols of the yeast two-hybrid method from Invitrogen Co., Ltd. and Clontech Co., Ltd.

The library vectors were recovered from the yeast colonies selected as described above, and the nucleotide sequences were analyzed using the DNA sequencer (the same as above). The nucleotide sequences are shown in SEQ ID NOS: 33 to 63 in the Sequence Listing.

The polypeptide obtained as described above (in a state of being inserted in the pB42AD vector (manufactured by Clontech Co., Ltd.) by the screening) was excised from the vector with the restriction enzymes EcoRI and XhoI. Then, the resulting fragments were subjected to a ligation reaction, using the Ligation Kit ver. 2 (manufactured by Takara Shuzo Co., Ltd.), with the pGBKT7 vector (manufactured by Clontech Co. , Ltd. ) which had been digested with the restriction enzymes EcoRI and SalI, thereby, the polypeptide-incorporated pGBKT7 vector was obtained. Similarly, the RAGECD fragments were excised from the above-described pHybLex / Zeo-RAGECD vector with the restriction enzymes EcoRI and XhoI, followed by being ligated to a pGADGH vector (manufactured by Clontech Co., Ltd.) which had been digested with the restriction enzymes EcoRI and SalI, thereby a pGBK T7 - RAGECD vector was obtained.

The pGBKT7-polypeptide vector was introduced into a yeast strain AH109 (manufactured by Clontech Co., Ltd.), and the pGADT7-RAGECD vector was introduced into a yeast strain Y187 (manufactured by Clontech Co., Ltd.), by using the lithium acetate method (see page 20 of "Yeast Protocols Handbook" attached to the kit of Clontech Co., Ltd.).

The plasmid-introduced yeasts were subjected to selection on a tryptophan-deficient SD medium or on a leucine-deficient SD medium, and selected strains were then conjugated with the following combinations (see page 44 of "Yeast Protocols Handbook" attached to the kit of Clontech Co. , Ltd.). Subsequently, the yeast in which two kinds of plasmids had been introduced was selected.
1: pGBKT7 - polynucleotide of SEQ ID NO: 43 + pGADT7 - idle vector
2. pGBKT7 - polynucleotide of SEQ ID NO: 44 + pGADT7 - idle vector
3. pGBKT7 - polynucleotide of SEQ ID NO: 61 + pGADT7 - idle vector
4. pGBKT7 - polynucleotide of SEQ ID NO: 62 + pGADT7 - idle vector
5. pGBKT7 - polynucleotide of SEQ ID NO: 43 + pGADT7 - RAGECD vector
6. pGBKT7 - polynucleotide of SEQ ID NO: 44 + pGADT7 - RAGECD vector
7. pGBKT7 - polynucleotide of SEQ ID NO: 61 + pGADT7 - RAGECD vector
8. pGBKT7 - polynucleotide of SEQ ID NO: 62 + pGADT7 - RAGECD vector

As a result of the growth test of the above strains on the SD medium lacking in tryptophan, leucine, histidine and adenine, strains 1, 2, 3, and 4 were not grown, while strains 5, 6, 7, and 8 were grown.

Therefore, it was shown that the polynucleotides of SEQ ID NOS: 43, 44, 61, and 62 were specifically bind to the RAGECD fragments, respectively.

That is, it was found that the polypeptide translated from each of those polynucleotides binds to the cytoplasmic domain of RAGE.

The above procedures were carried out on the basis of protocols of MATCH MAKER GAL4 Yeast Two Hybrid System 3 (manufactured by Clontech Co., Ltd.).

### Example 2

### Cloning of protein which can interact with a cytoplasmic domain of RAGE by yeast two-hybrid method

A gene for a cytoplasmic domain for RAGE (SEQ ID NO: 65 in the Sequence Listing) (J. Biol. Chem 1992 Jul 25, 267 (21): 14998-5004) was amplified using synthetic oligonucleotides: 5'-ACGTGAATTCAGGCGGCAACGCCGAGGAG-3' (SEQ ID NO: 66 in the Sequence Listing) and 5'-CGATCTCGAGTCAAGGCCCTCCAGTACTACTC-3' (SEQ ID NO: 67 in the Sequence Listing), and was then digested with restriction enzymes EcoRI and XhoI. Also, a vector pGBKT7 (manufactured by Clontech Co., Ltd.) was digested with restriction enzymes EcoRI and Sal I. Subsequently, the both genes were subjected to a 14-hour ligation reaction using the Takara Ligation Kit ver. 2 (manufactured by Takara Shuzo Co., Ltd.) and used to transform *E*. *coli* strain DH5α (manufactured by Takara Shuzo Co., Ltd.), thereby colonies were obtained. Consequently, a vector (pGBKT7-RAGECD) in which the cytoplasmic domain of RAGE is introduced in the pGBKT7 was obtained. Using a DNA sequencer (manuf actured by Beckman Co . , Ltd.), the sequence of the cytoplasmic domain of RAGE was confirmed.

The vector pGBKT7-RAGECD obtained as described above was introduced into yeast strain AH109 attached to the kit MATCH MAKER described below. Then, a clone into which the PGBKT7-RAGECD had been introduced was selected.

Then, Pretransformated MATCHMAKER library Human Brain (manufactured by Clontech Co., Ltd.) was introduced into the above strain and 10⁶ colonies were screened. At the first screening, a histidine-deficient medium was used, and about 140 colonies were selected based on the expression of a histidine gene, which was a reporter of the interaction. At the second screening, 40 colonies were selected on an adenine-deficient medium based on the expression of adenine, which was another reporter of the interaction.

Finally, 7 clones each of which had been confirmed to surely have two kinds of reporter activities were selected, the library vectors were recovered, and the nucleotide sequences were then analyzed using the DNA sequencer (the same as above). The nucleotide sequences are shown in SEQ ID NOS: 87 to 93 in the Sequence Listing.

The above procedures were carried out on the basis of protocols of the yeast two-hybrid method from Invitrogen Co., Ltd. and Clontech Co., Ltd.

### Example 3

### Functional analysis on the cloned polypeptide

Because the polypeptide of SEQ ID NO: 62 was a part of protein kinase C zeta (PKC zeta), the full-length PKC zeta was cloned in pcDNA 3.1 (+) (manufactured by Invitrogen Co., Ltd.) using the PCR method, and pcDNA 3.1 (+) - PKC zeta vector was obtained.

Using TransIT-LT1 (manufactured by Takara Shuzo Co., Ltd.), pNFkB-luc (manufactured by Stratagene Co., Ltd) was introduced into rat C6 glioma cells (ATCC CCL-107). The cells were cultured in a medium with the addition of geneticin (manufactured by Nacalai Tesque, Inc.) to obtain the drug-resistant cells. Thus, stably expressing cells were obtained.

Using the TransIT-LT1 (manufactured by Takara Shuzo Co., Ltd.), pcDNA 3.1 (+) and pcDNA 3.1 (+) - PKC zeta plasmids were introduced into the cells, respectively. After 48 hours, the medium was replaced with a serum-free DMEM medium (manufactured by SIGMA Co., Ltd.). After an additional 12 hours, the cells were stimulated for 6 hours with carboxy methyl lysine (CML) which had been prepared by incubating 1.7 g of BSA (manufactured by SIGMA Co., Ltd.) and 0.36 g of glyoxylic acid (manufactured by OHSIGMA Co., Ltd.) at 36°C for 24 hours in the presence of a sodium cyano borohydride (manufactured by SIGMA Co., Ltd.) as a catalyst. After the stimulation, a luciferase assay was performed using Bright-Glo (manufactured by Promega Co. , Ltd.), in which the cells were measured with an ARVOsx plate reader (manufactured by Wallac Co. , Ltd.). Consequently, it was found that the introduction of pcDNA 3.1 (+) - PKC zeta significantly increased the NFkB activity (Fig. 1) as compared to the introduction of an empty vector pcDNA 3.1.

In the case of diseases such as a diabetic complication in which RAGE may be involved, the activation of NFkB by AGE was known in the art. However, the intermediate pathway thereof had been unclear.

From this experiment, it was found that the polypeptide capable of interacting with the intracellular domain of the RAGE increases NFkB activation due to AGE stimulation. Therefore, it was suggested that the polynucleotide of SEQ ID NO: 62 would mediate the downstream signal of RAGE.

### Industrial Applicability

According to the present invention, a novel polypeptide involved in the signal transduction of RAGE is obtained. In addition, the polynucleotide encoding the polypeptide of the present invention can be used as a genetic source to be applied in a gene therapy. Furthermore, the polypeptide of the present invention is capable of providing a method of screening a substance useful for treating diseases involved in the signal transduction from RAGE through activation of NFκB, a pharmaceutical composition obtainable by the screening method, and a diagnostic agent for those diseases.

Note that, the present application has been filed claiming a priority of Japanese Patent Application No. 2001-219122.

## Claims

1. A polypeptide, which is a substantially purified polypeptide, wherein the polypeptide has following characteristics:
(a) the polypeptide directly or indirectly binds to a cytoplasmic domain of the receptor for an advanced glycation endproduct (AGE); and
(b) the polypeptide inhibits a signal transduction from binding of a ligand to the receptor for advanced glycation endproduct (AGE) through activation of NFκB.

2. The polypeptide according to claim 1, which comprises the amino acid sequence selected from SEQ ID NOS: 1 to 32 and SEQ ID NOS: 67 to 79 in the Sequence Listing or said amino acid sequence including deletion, substitution, or addition of one or several amino acids.

3. The polypeptide according to claim 1 or 2, which comprises the amino acid sequence selected from SEQ ID NOS: 11, 12, 29, and 30 in the Sequence Listing or said amino acid sequence including deletion, substitution, or addition of one or several amino acids.

4. The polypeptide according to claim 1, which comprises the amino acid sequence selected from SEQ ID NOS: 80 to 86 in the Sequence Listing or said amino acid sequence including deletion, substitution, or addition of one or several amino acids.

5. A polynucleotide, which encodes the polypeptide according to any one of claims 1 to 4.

6. The polynucleotide according to claim 5, comprising the nucleotide sequence represented by one selected from SEQ ID NOS: 33 to 63 or a nucleotide sequence which is hybridizable with the polynucleotide represented by one selected from SEQ ID NOS: 33 to 63 under a stringent condition.

7. The polynucleotide according to claim 5 or 6, comprising the nucleotide sequence represented by one selected from SEQ ID NOS: 43, 44, 61, and 62 or a nucleotide sequence which is hybridizable with the polynucleotide represented by one selected from SEQ ID NOS: 43, 44, 61, and 62 under a stringent condition.

8. The polynucleotide according to claim 5, comprising the nucleotide sequence represented by one selected from SEQ ID NOS: 87 to 93 or a nucleotide sequence which is hybridizable with the polynucleotide represented by one selected from SEQ ID NOS: 87 to 93 under a stringent condition.

9. A pharmaceutical for a gene therapy, comprising the polynucleotide according to any one of claims 5 to 8 and a vector which is expressible in an animal.

10. The pharmaceutical for a gene therapy according to claim 9, which is used for treating a disease selected from diabetes, a diabetic complication, Alzheimer's Disease, dialysis amyloidosis, a cancer, a periodontal disease, and an aging-related disease.

11. A vector, comprising the polynucleotide according to any one of claims 5 to 8.

12. A microorganism or cell, which is transformed with the vector according to claim 11.

13. A method for producing the polypeptide according to any one of claims 1 to 4, comprising:
culturing the microorganism or cell according to claim 12; and
isolating the polypeptide according to any one of claims 1 to 4 from the culture.

14. A method for screening a substance that inhibits or accelerates the biding of the polypeptide according to any one of claims 1 to 4 to a cytoplasmic domain of the receptor for advanced glycation endproduct (AGE), comprising:
placing a target of screening in a screening system that contains the cytoplasmic domain of the receptor for advanced glycation endproduct (AGE) and the polypeptide according to any one of claims 1 to 4; and
measuring a degree of inhibition or acceleration of the binding of the polypeptide according to any one of claims 1 to 4 to a cytoplasmic domain of the receptor for advanced glycation end product (AGE).

15. A method for screening a substance that enhances or inhibits a function of the polypeptide according to any one of claims 1 to 4 or a substance that increases or decreases an amount of the polypeptide according to any one of claims 1 to 4, comprising:
placing a target of screening in a screening system that contains the polypeptides according to any one of claims 1 to 4; and
measuring a degree of enhancement or inhibition of the function of the polypeptide according to any one of claims 1 to 4 or a degree of increase or decrease of the amount of the polypeptide according to any one of claims 1 to 4.

16. A compound, which is obtainable by the screening method according to claim 14 or 15.

17. The compound according to claim 16, which has following characteristics:
(a) the compound directly or indirectly binds to a cytoplasmic domain of the receptor for advanced glycation endproduct (AGE); and
(b) the compound inhibits a signal transduction from binding of a ligand to the receptor for advanced glycation endproduct (AGE) through activation of NFκB.

18. A pharmaceutical composition, comprising the substance selected from the group consisting of the compound according to claim 16 or 17, a salt thereof, a hydrate thereof, and a solvate thereof.

19. The pharmaceutical composition according to claim 18, which is used for treating a disease selected from diabetes, a diabetic complication, Alzheimer's Disease, dialysis amyloidosis, a cancer, a periodontal disease, and an aging-related disease.

20. An antibody, which can specifically bind to the polypeptide according to any one of claims 1 to 4.

21. A polynucleotide, which is represented by a nucleotide sequence having at least 20 sequential nucleotides in any of the sequences of SEQ ID NOS: 33 to 63 in the Sequence Listing, a sequence complementary to the nucleotide sequence, or a nucleotide sequence which is hybridizable with the nucleotide sequence under a stringent condition.

22. A polynucleotide, which is represented by a nucleotide sequence having at least 20 sequential nucleotides in any of the sequences of SEQ ID NOS: 43, 44, 61, and 62 in the Sequence Listing, a sequence complimentary to the nucleotide sequence, or a nucleotide sequence which is hybridizable with the nucleotide sequence under a stringent condition.

23. A polynucleotide, which is represented by a nucleotide sequence having at least 20 sequential nucleotides in any of the sequences of SEQ ID NOS: 87 to 93 in the Sequence Listing, a sequence complimentary to the nucleotide sequence, and a nucleotide sequence which is hybridizable with the nucleotide sequence under a stringent condition.

24. A probe, comprising a label and the polynucleotide according to any one of claims 21 to 23.

25. A reagent for diagnosis, comprising the probe according to claim 24.

26. The reagent for diagnosis according to claim 25, which is used for a diagnosis of diabetes, a diabetic complication, Alzheimer's Disease, dialysis amyloidosis, a cancer, a periodontal disease, and an aging-related disease.


SEQUENCE LISTING

<110> MITSUBISHI PHARMA CORPORATION

<120> POLYPEPTIDE INVOLVED IN SIGNAL TRANSDUCTION OF RECEPTOR FOR ADVANCED GLYCATION
ENDPRODUCT

<130> 02029W00

<140>
<141>

<150> JP2001-219122
<151> 2001-07-19

<160> 93

<170> PatentIn Ver. 2.1

<210> 1
<211> 8
<212> PRT
<213> Homo sapiens

<400> 1
Val Val Asp Met Arg Arg Tyr Phe
  1               5

<210> 2
<211> 132
<212> PRT
<213> Homo sapiens

<400> 2
Met Val Thr Ala Gly His Ala Cys Thr Lys Lys Tyr Thr Pro Glu Gln

1                   5                   10                  15

Val Ala Met Ala Thr Val Thr Ala Leu His Arg Thr Val Pro Ala Ala
                20                  25                  30

Val Pro Gly Ile Cys Phe Leu Ser Gly Gly Met Ser Glu Glu Asp Ala
            35                  40                  45

Thr Leu Asn Leu Asn Ala Ile Asn Leu Cys Pro Leu Pro Lys Pro Trp
        50                  55                  60

Lys Leu Ser Phe Ser Tyr Gly Arg Ala Leu Gln Ala Ser Ala Leu Ala
65                  70                  75                  80

Ala Trp Gly Gly Lys Ala Ala Asn Lys Glu Ala Thr Gln Glu Ala Phe
                85                  90                  95

Met Lys Arg Ala Met Ala Asn Cys Gln Ala Ala Lys Gly Gln Tyr Val
            100                 105                 110

His Thr Gly Ser Ser Gly Ala Ala Ser Thr Gln Ser Leu Phe Thr Ala
        115                 120                 125

Cys Tyr Thr Tyr
    130

<210> 3
<211> 5
<212> PRT
<213> Homo sapiens

<400> 3
Ile Ile Ile Gly Ser
1               5

<210> 4
<211> 5
<212> PRT
<213> Homo sapiens

<400> 4
Ser Leu Phe Leu Met
1               5

<210> 5
<211> 128
<212> PRT
<213> Homo sapiens

<400> 5
Leu Val Ala Leu Pro Ala Thr Pro Leu Thr Pro Ala Phe Pro Pro Pro
1               5                   10                  15

Ser Phe Asp Gln Arg Ser Ala Glu Thr Leu Glu Val Arg Lys Leu Asp
            20                  25                  30

Thr His Pro Asp Arg Thr Gly Val Leu Arg Gly Val Ala Gly Gly Phe
        35                  40                  45

Gly Leu Asp Glu Phe Pro Pro Lys Cys Arg Arg Ser Gln Ala Arg Ala
    50                  55                  60

Gln Lys Gln Arg Met Gly Lys Ser Met Gln His Ser Val Tyr Leu Ile
65                  70                  75                  80

Leu Thr Arg Gly Asn His Lys His Arg Lys Pro Gln Leu Asp Met Asp
                85                  90                  95

Tyr Arg Val Leu Glu Lys Ser Val Phe His Gly Val Cys Leu His Leu

            100                 105                 110

Leu Thr Ser Gln Val Leu Lys Gln Lys Thr Lys Ala Met Ile Leu Met
        115                 120                 125

<210> 6
<211> 25
<212> PRT
<213> Homo sapiens

<400> 6
Arg Lys Ile Asn Pro Leu Ile Lys Leu Ile Asn His Ser Phe Ile Asp
1               5                   10                  15

Leu Pro Thr Pro Ser Asn Ile Ser Ala
            20                  25

<210> 7
<211> 19
<212> PRT
<213> Homo sapiens

<400> 7
Ser Val Arg Gly Ser Val Val Gly Ser Thr Leu Thr Pro Leu Ala Cys
1               5                   10                  15

Pro Asp Pro

<210> 8
<211> 15
<212> PRT
<213> Homo sapiens

<400> 8
Gln Leu Cys Trp Leu Asp Trp Leu Pro Cys Ser Trp Pro Arg Thr
1               5                   10                  15

<210> 9
<211> 5
<212> PRT
<213> Homo sapiens

<400> 9
Ser Phe Gly Ser Leu
1               5

<210> 10
<211> 7
<212> PRT
<213> Homo sapiens

<400> 10
Arg Lys Asn Lys Leu Arg Gln
1               5

<210> 11
<211> 21
<212> PRT
<213> Homo sapiens

<400> 11
Leu Arg Glu Lys Met Thr Gly Tyr Ile Gln Ser Asp Met Ala Lys Ser
1               5                   10                  15

Leu Lys Val Leu Met

            20

<210> 12
<211> 132
<212> PRT
<213> Homo sapiens

<400> 12
Arg Val Thr Ile Arg Lys Ser Lys Asn Ile Leu Phe Val Ile Thr Lys
1               5                   10                  15

Pro Asp Val Tyr Lys Ser Pro Ala Ser Asp Thr Tyr Ile Val Phe Gly
            20                  25                  30

Glu Ala Lys Ile Glu Asp Leu Ser Gln Gln Ala Gln Leu Ala Ala Ala
        35                  40                  45

Glu Lys Phe Lys Val Gln Gly Glu Ala Val Ser Asn Ile Gln Glu Asn
    50                  55                  60

Thr Gln Thr Pro Thr Val Gln Glu Glu Ser Glu Glu Glu Glu Val Asp
65                  70                  75                  80

Glu Thr Gly Val Glu Val Lys Asp Ile Glu Leu Val Met Ser Gln Ala
                85                  90                  95

Asn Val Ser Arg Ala Lys Ala Val Arg Ala Leu Lys Asn Asn Ser Asn
            100                 105                 110

Asp Ile Val Asn Ala Ile Met Glu Leu Thr Met Xaa Pro Tyr Gly Ser
        115                 120                 125

Asn Phe Phe Trp
    130

<210> 13
<211> 23
<212> PRT
<213> Homo sapiens

<400> 13
Lys Gly Lys Asn Asp Trp Leu Tyr Ser Val Arg Tyr Gly Lys Lys Ser
1               5                   10                  15

Gln Gly Val Asn Val Asn Asp
            20

<210> 14
<211> 66
<212> PRT
<213> Homo sapiens

<400> 14
Glu His Met Val Ile Thr Asp Arg Ile Glu Asn Ile Asp His Leu Gly
1               5                   10                  15

Phe Phe Ile Tyr Arg Leu Cys His Asp Lys Glu Thr Tyr Lys Leu Gln
            20                  25                  30

Arg Arg Glu Thr Ile Lys Gly Ile Gln Lys Arg Glu Ala Ser Asn Cys
        35                  40                  45

Phe Ala Ile Arg His Phe Glu Asn Lys Phe Ala Val Glu Thr Leu Ile
    50                  55                  60

Cys Ser
65

<210> 15
<211> 180
<212> PRT
<213> Homo sapiens

<400> 15
Gly Gly Glu Glu Pro Ala Glu Glu Asp Ser Glu Asp Trp Cys Val Pro
1               5                   10                  15

Cys Ser Asp Glu Glu Val Glu Leu Pro Ala Asp Gly Gln Pro Trp Met
            20                  25                  30

Pro Pro Pro Ser Glu Ile Gln Arg Leu Tyr Glu Leu Leu Ala Ala His
        35                  40                  45

Gly Thr Leu Glu Leu Gln Ala Glu Ile Leu Pro Arg Arg Pro Pro Thr
    50                  55                  60

Pro Glu Ala Gln Ser Glu Glu Glu Arg Ser Asp Glu Glu Pro Glu Ala
65                  70                  75                  80

Lys Glu Glu Glu Glu Glu Lys Pro His Met Pro Thr Glu Phe Asp Phe
                85                  90                  95

Asp Asp Glu Pro Val Thr Pro Lys Asp Ser Leu Ile Asp Arg Arg Arg
            100                 105                 110

Thr Pro Gly Ser Ser Ala Arg Ser Gln Lys Arg Glu Ala Arg Leu Asp
        115                 120                 125

Lys Val Leu Ser Asp Met Lys Arg His Lys Lys Leu Glu Glu Gln Ile
    130                 135                 140

Leu Arg Thr Gly Arg Asp Leu Phe Ser Leu Asp Ser Glu Asp Pro Ser
145                 150                 155                 160

Pro Ala Ser Pro Pro Leu Arg Ser Ser Gly Ser Ser Leu Phe Pro Arg
                165                 170                 175

Gln Arg Lys Tyr
            180

<210> 16
<211> 19
<212> PRT
<213> Homo sapiens

<400> 16
Arg Thr Val Ser Ser Ile Asn Gly Val Gly Lys Thr Gly Tyr Pro Tyr
1               5                   10                  15

Ala Lys Glu

<210> 17
<211> 19
<212> PRT
<213> Homo sapiens

<400> 17
Asn Leu Gly Leu Leu Phe Ile Leu Ala Thr Ser Ser Leu Ala Val Tyr
1               5                   10                  15

Ser Ile Leu

<210> 18
<211> 30
<212> PRT

<213> Homo sapiens

<400> 18
Lys Ser Met Lys Asn Asn Pro Val Ile Val Phe Ala Thr Lys Gly Lys
1               5                   10                  15

Gln Cys Lys Met Thr Tyr Ser Ile Ser Ser Cys Ser Asn Tyr
            20                  25                  30

<210> 19
<211> 163
<212> PRT
<213> Homo sapiens

<400> 19

<210> 20
<211> 105
<212> PRT
<213> Homo sapiens

<400> 20
Lys Asn Ala Leu Ala His Phe Leu Pro Gln Gly Thr Pro Thr Pro Leu
1               5                   10                  15

Ile Pro Ile Leu Val Ile Ile Glu Thr Ile Ser Leu Leu Ile Gln Pro
            20                  25                  30

Ile Ala Leu Ala Val Arg Leu Thr Ala Asn Ile Thr Ala Gly His Leu
        35                  40                  45

Leu Met His Leu Ile Gly Ser Ala Thr Leu Ala Ile Ser Thr Ile Asn
    50                  55                  60

Leu Pro Ser Thr Leu Ile Ile Phe Thr Ile Leu Ile Leu Leu Thr Ile
65                  70                  75                  80

Leu Glu Ile Ala Val Ala Leu Ile Gln Ala Tyr Val Phe Thr Leu Leu

                85                  90                  95

Val Ser Leu Tyr Leu His Asp Asn Thr
            100                 105

<210> 21
<211> 117
<212> PRT
<213> Homo sapiens

<400> 21
Ala Arg Pro Ile Leu Arg Ile Ile Ser Leu Leu Arg Asn Leu Lys His
1               5                   10                  15

Arg His Tyr Pro Pro Ala Cys Asn Tyr Ser Asn Ser Leu His Arg Leu
            20                  25                  30

Cys Pro Pro Val Arg Pro Asn Ile Ile Leu Arg Gly His Ser Asn Tyr
        35                  40                  45

Lys Leu Thr Ile Arg His Pro Ile His Trp Asp Arg Pro Ser Ser Met
    50                  55                  60

Asn Leu Arg Arg Leu Leu Ser Arg Gln Ser His Pro His Thr Ile Leu
65                  70                  75                  80

Tyr Leu Ser Leu His Leu Ala Leu His Tyr Cys Ser Pro Ser Ser Thr
                85                  90                  95

Pro Pro Pro Ile Leu Ala Arg Asn Gly Ile Lys Gln Pro Pro Arg Asn
            100                 105                 110

His Leu Pro Phe Arg
        115

<210> 22
<211> 24
<212> PRT
<213> Homo sapiens

<400> 22
Glu Pro Ser Gly Ser Cys Ala Asn Arg His Gln Tyr Gln Leu Cys Glu
1               5                   10                  15

Leu Gly Cys Glu Cys Thr Arg Ala
            20

<210> 23
<211> 520
<212> PRT
<213> Homo sapiens

<400> 23
Pro Thr Asp Ser Thr Met Leu Lys Lys Phe Asp Lys Lys Asp Glu Glu
1               5                   10                  15

Ser Gly Gly Gly Ser Asn Pro Phe Gln His Leu Glu Lys Ser Ala Val
            20                  25                  30

Leu Gln Glu Ala Arg Val Phe Asn Glu Thr Pro Ile Asn Pro Arg Lys
        35                  40                  45

Cys Ala His Ile Leu Thr Lys Ile Leu Tyr Leu Ile Asn Gln Gly Glu
    50                  55                  60

His Leu Gly Thr Thr Glu Ala Thr Glu Ala Phe Phe Ala Met Thr Lys
65                  70                  75                  80

Leu Phe Gln Ser Asn Asp Pro Thr Leu Arg Arg Met Cys Tyr Leu Thr

                85                  90                  95

Ile Lys Glu Met Ser Cys Ile Ala Glu Asp Val Ile Ile Val Thr Ser
            100                 105                 110

Ser Leu Thr Lys Asp Met Thr Gly Lys Glu Asp Asn Tyr Arg Gly Pro
        115                 120                 125

Ala Val Arg Ala Leu Cys Gln Ile Thr Asp Ser Thr Met Leu Gln Ala
    130                 135                 140

Ile Glu Arg Tyr Met Lys Gln Ala Ile Val Asp Lys Val Pro Ser Val
145                 150                 155                 160

Ser Ser Ser Ala Leu Val Ser Ser Leu His Leu Leu Lys Cys Ser Phe
                165                 170                 175

Asp Val Val Lys Arg Trp Val Asn Glu Ala Gln Glu Ala Ala Ser Ser
            180                 185                 190

Asp Asn Ile Met Val Gln Tyr His Ala Leu Gly Leu Leu Tyr His Val
        195                 200                 205

Arg Lys Asn Asp Arg Leu Ala Val Asn Lys Met Ile Ser Lys Val Thr
    210                 215                 220

Arg His Gly Leu Lys Ser Pro Phe Ala Tyr Cys Met Met Ile Arg Val
225                 230                 235                 240

Ala Ser Lys Gln Leu Glu Glu Glu Asp Gly Ser Arg Asp Ser Pro Leu
                245                 250                 255

Phe Asp Phe Ile Glu Ser Cys Leu Arg Asn Lys His Glu Met Val Val
            260                 265                 270

Tyr Glu Ala Ala Ser Ala Ile Val Asn Leu Pro Gly Cys Ser Ala Lys

275                 280                 285

Glu Leu Ala Pro Ala Val Ser Val Leu Gln Leu Phe Cys Ser Ser Pro
    290                 295                 300

Lys Ala Ala Leu Arg Tyr Ala Ala Val Arg Thr Leu Asn Lys Val Ala
305                 310                 315                 320

Met Lys His Pro Ser Ala Val Thr Ala Cys Asn Leu Asp Leu Glu Asn
                325                 330                 335

Leu Val Thr Asp Ser Asn Arg Ser Ile Ala Thr Leu Ala Ile Thr Thr
            340                 345                 350

Leu Leu Lys Thr Gly Ser Glu Ser Ser Ile Asp Arg Leu Met Lys Gln
        355                 360                 365

Ile Ser Ser Phe Met Ser Glu Ile Ser Asp Glu Phe Lys Val Val Val
    370                 375                 380

Val Gln Ala Ile Ser Ala Leu Cys Gln Lys Tyr Pro Arg Lys His Ala
385                 390                 395                 400

Val Leu Met Asn Phe Leu Phe Thr Met Leu Arg Glu Glu Gly Gly Phe
                405                 410                 415

Glu Tyr Lys Arg Ala Ile Val Asp Cys Ile Ile Ser Ile Ile Glu Glu
            420                 425                 430

Asn Ser Glu Ser Lys Glu Thr Gly Leu Ser His Leu Cys Glu Phe Ile
        435                 440                 445

Glu Asp Cys Glu Phe Thr Val Leu Ala Thr Arg Ile Leu His Leu Leu
    450                 455                 460

Gly Gln Glu Gly Pro Lys Thr Thr Asn Pro Ser Lys Tyr Ile Arg Phe

465                 470                 475                 480

Ile Tyr Asn Arg Val Val Leu Glu His Glu Glu Val Arg Ala Gly Ala
                485                 490                 495

Val Ser Ala Leu Ala Lys Phe Gly Ala Gln Asn Glu Glu Met Leu Pro
            500                 505                 510

Ser Ile Leu Val Leu Leu Lys Arg
        515                 520

<210> 24
<211> 21
<212> PRT
<213> Homo sapiens

<400> 24
Pro Gln Thr His Ser Thr Leu Leu Pro Asp Asn Leu Ser Gln Thr Ile
 1               5                  10                  15

Tyr Pro Asn Lys Val
            20

<210> 25
<211> 46
<212> PRT
<213> Homo sapiens

<400> 25
His Asn Lys Gln Thr Trp Ser Ile Leu Gly Leu Leu His Ala Leu Pro
 1               5                  10                  15

Glu Pro Val Pro Ala Ala Gly Gly Ser Pro Gly Glu Arg Leu Trp Pro
            20                  25                  30

Leu Gln Leu Leu Arg Glu Cys Ala Arg Gln Thr Arg Gln Ile
        35                  40                  45

<210> 26
<211> 34
<212> PRT
<213> Homo sapiens

<400> 26
Asn Thr Lys Ile Cys Gln Ala Trp Trp Arg Thr Pro Ile Ile Pro Ala
 1               5                  10                  15

Thr Gly Glu Ala Glu Ala Gly Glu Pro Leu Glu Pro Arg Arg Gln Arg
            20                  25                  30

Leu Gln

<210> 27
<211> 180
<212> PRT
<213> Homo sapiens

<400> 27
Gly Leu Leu Lys Val Val Phe Val Val Phe Ala Ser Leu Cys Ala Trp
 1               5                  10                  15

Tyr Ser Gly Tyr Leu Leu Ala Glu Leu Ile Pro Asp Ala Pro Leu Ser
            20                  25                  30

Ser Ala Ala Tyr Ser Ile Arg Ser Ile Gly Glu Arg Pro Val Leu Lys
        35                  40                  45

Gly Glu Cys Arg Ala Trp Gly Arg Arg Leu Pro Ser Trp Leu Val Cys
    50                  55                  60

Arg Gly Arg Ser Gly Gly Phe Cys Pro Ser Trp Arg Leu Gly Gly Pro
65                  70                  75                  80

Asp Gly Phe Ile Ser Gly Arg Arg Arg Arg Glu Ala Phe Cys Ser Tyr
                85                  90                  95

Ser Phe Arg Ser His Ala Ile Pro Arg His Val Pro Leu Leu Thr Pro
            100                 105                 110

Cys Glu Thr Arg Thr Val Trp Leu Leu Val Thr Ser Thr Asn Leu Gln
        115                 120                 125

Pro Asp Leu Glu Leu Cys Ser Phe Arg Pro Asp Leu Gly Phe Leu Pro
    130                 135                 140

Trp Glu Ala Gln Glu Gly Pro Gly Ser Glu Ile Pro Asn His Phe Lys
145                 150                 155                 160

Val Ser Val Gly Leu Lys Ser Cys Trp Lys Ile Phe Cys Lys Val Leu
                165                 170                 175

Gly Ser Lys Ser
            180

<210> 28
<211> 34
<212> PRT
<213> Homo sapiens

<400> 28
Lys Lys Lys Ser Thr Trp Val Gln Trp Leu Thr Pro Val Ile Leu Ala
1               5                   10                  15

Phe Gly Glu Thr Lys Val Gly Gly Ser Leu Glu Pro Gly Arg Ser Arg
            20                  25                  30

Leu Gln

<210> 29
<211> 7
<212> PRT
<213> Homo sapiens

<400> 29
Arg Ser Cys His Asp Leu Asn
1               5

<210> 30
<211> 237
<212> PRT
<213> Homo sapiens

<400> 30
Tyr Ala Ala Glu Ile Cys Ile Ala Leu Asn Phe Leu His Glu Arg Gly
1               5                   10                  15

Ile Ile Tyr Arg Asp Leu Lys Leu Asp Asn Val Leu Leu Asp Ala Asp
            20                  25                  30

Gly His Ile Lys Leu Thr Asp Tyr Gly Met Cys Lys Glu Gly Leu Gly
        35                  40                  45

Pro Gly Asp Thr Thr Ser Thr Phe Cys Gly Thr Pro Asn Tyr Ile Ala
    50                  55                  60

Pro Glu Ile Leu Arg Gly Glu Glu Tyr Gly Phe Ser Val Asp Trp Trp
65                  70                  75                  80

Ala Leu Gly Val Leu Met Phe Glu Met Met Ala Gly Arg Ser Pro Phe
                85                  90                  95

Asp Ile Ile Thr Asp Asn Pro Asp Met Asn Thr Glu Asp Tyr Leu Phe
            100                 105                 110

Gln Val Ile Leu Glu Lys Pro Ile Arg Ile Pro Arg Phe Leu Ser Val
        115                 120                 125

Lys Ala Ser His Val Leu Lys Gly Phe Leu Asn Lys Asp Pro Lys Glu
    130                 135                 140

Arg Leu Gly Cys Arg Pro Gln Thr Gly Phe Ser Asp Ile Lys Ser His
145                 150                 155                 160

Ala Phe Phe Arg Ser Ile Asp Trp Asp Leu Leu Glu Lys Lys Gln Ala
                165                 170                 175

Leu Pro Pro Phe Gln Pro Gln Ile Thr Asp Asp Tyr Gly Leu Asp Asn
            180                 185                 190

Phe Asp Thr Gln Phe Thr Ser Glu Pro Val Gln Leu Thr Pro Asp Asp
        195                 200                 205

Glu Asp Ala Ile Lys Arg Ile Asp Gln Ser Glu Phe Glu Gly Phe Glu
    210                 215                 220

Tyr Ile Asn Pro Leu Leu Leu Ser Thr Glu Glu Ser Val
225                 230                 235

<210> 31
<211> 54

<212> PRT
<213> Homo sapiens

<400> 31
Phe Trp Ile Leu Pro Ser Ile Ser Arg Pro Leu Arg Ala Arg His Phe
1               5                   10                  15

Arg Ala Gly Gln Asn Leu Cys Arg Ser Arg Cys Gly Phe Ala Gly Asp
            20                  25                  30

Gln Gly Gly Asp Glu Lys Gly Arg Gly Gly Ala Pro Ala Gln Glu Arg
        35                  40                  45

Gly Cys Gly Ala Pro Gly
    50

<210> 32
<211> 26
<212> PRT
<213> Homo sapiens

<400> 32
Lys Lys Lys Lys Tyr Leu Gly Thr Val Ala His Thr Cys Asn Pro Ser
1               5                   10                  15

Ile Trp Gly Asp Gln Gly Gly Arg Ile Thr
            20                  25

<210> 33
<211> 1145
<212> DNA
<213> Homo sapiens

<400> 33

gttgtagata tgcggcgtta tttctgaggg ctctgttctg ttccattgat ctatatctct 60
gtttcggtac cagtaccatg ctgttactgt agccttgtag tatagtttga aatcaggtag 120
cgtgatgcct ccagctttgt tctttcggct taggattgac ttggcaatgc gggctctttt 180
ttggttccat atgaacttta aagtagtttt ttccaattct gtgaagaaag tgattggtag 240
cttgatgggg atggcattga atctataaat taccttaggc agtatggcca ttttcacgat 300
attgattctt cctacccatg agcatggaat gttcttccat ttctttgtat cctcttttat 360
ttcattgagc agtggtttgt agttctcctt gatgaggtcc ttcacaaaaa ctaacaaaca 420
gaaaggacgt gacagcccag agtttttaaa gctgacactt gagttgggtt tttctctttc 480
tttaccactg taaaaaaata atgaatgaga attgggtgct gggttttgtt ttgttgtttt 540
agtggtggca gggaagctgg ggatgcgggt gattaggctg agtattacag aggtagcagg 600
agtcccatag caccaagttc catcttcttg ttacccattc cagaggcccc aaacctacct 660
agaggctttg ggatcaggag gcttgagctt ggattctggc tctgccacct ccttacctag 720
aacccctttg taagtcactg agtttgtgag tctcaacgtc gtcattacaa aagtacagaa 780
acaccaacct tctctttcat ggtgggtagt gtggtagtac tggactctgg atgagttata 840
ctttaaaagc tatcaactgt atatcatgta atcctgcccc tacaggactg attcactaac 900
aagataactg tttaaaagaa agaaaaagag agcggggcgc agtggctcac acctgtaatc 960
ccagcacttt gggaggctga ggcgggtgga tcatttgagg tcaggagttt gaggccagcc 1020
tggccaacat agtgaaaccc tgtctctacc agaaatacaa aaaaattagc cgggtgtggt 1080
agtgcatgcc tataatccca gctacttggg aggctgaggc aggagaatca cttgaacccg 1140
ggagg 1145

<210> 34
<211> 929
<212> DNA
<213> Homo sapiens

<400> 34
   aacactgcca gtatgttact gagaaggtcc tggctgctgt ctacaaggcc ctgaatgacc 60
   atcatgttta cctggagggc accctgctaa agcccaacat ggtgactgct ggacatgcct 120
   gcaccaagaa gtatactcca gaacaagtag ctatggccac cgtaacagct ctccaccgta 180
   ctgttcctgc agctgttcct ggcatctgct tttgtctggt tggcatgagt gaagaggatg 240
   ccactctcaa cctcaatgct atcaaccttt gccctctacc aaagccctgg aaactaagtt 300
   tctcttatgg acgggccctg caggccagtg cactggctgc ctggggtggc aaggctgcaa 360
   acaaggaggc aacccaggag gcttttatga agcgggccat ggctaactgc caggcggcca 420
   aaggacagta tgttcacacg ggttcttctg gggctgcttc cacccagtcg ctcttcacag 480
   cctgctatac ctactagggt ccaatgcccg ccagcctagc tccagtgctt ctagtaggag 540
   ggctgaaagg gagcaacttt tcctctaatc ctggaaattc gacacaatta gatttgaact 600
   gctggaaata caacacatgt taaatcttaa gtacaagggg gaaaaaataa atcagttatt 660
   gaaacataaa aatgaatacc aaggacctga tcaaatttca cacagcagtt tccttgcaac 720
   actttcagct ccccatgctc cagaataccc acccaagaaa ataataggct ttaaaacaat 780
   atcggctcct catccaaaga acaactgctg attgaaacac ctcattagct gagtgtagag 840
   aagtgcatct tatgaaacag tcttagcagt ggtaggttgg gaaggagata gctgcaacca 900
   aaaaagaaat aaatattcta taaaccttc 929

<210> 35
<211> 479
<212> DNA
<213> Homo sapiens

<400> 35
cataatcata ggtagctagg ttataaacta tttaaagaca agatcacgtg ataagcttat 60
aatcttctca taattcccct tacttagcat tgtgttagac atactaatag gtgcacagtg 120
aaatacttat tgttgattgt ttaaaaataa agttttagaa aaccttttca aaagtcagag 180
tttaggccag gggcacaggc tgacacctat aatcccagca ctttgggagg ccagggcggg 240
cagatcactt gggtcaagag ttcaaggcca gcctggccaa catggcaaaa ccccatctct 300
actaaataaa atacaaaaat tatccaggca tggtggtgca tgcctgtaat cccagctact 360
tggaggctga ggcatgagaa ttgcttgaac ctgggaggca gaggttgcag tgagctgaga 420
tcgccccact gcaatccagc ctgggagaca taattcaaat ctattttggt cttatatct 479

<210> 36
<211> 253
<212> DNA
<213> Homo sapiens

<400> 36
ctctcttttt ctaatgtaaa tgttgtgtac aatagtttta tttgattaag cttcaggact 60
gttttgtaaa gcgaggtggg accgatgtgg cacacgccag ctgcggtttc ccggagcgtg 120
gagaggcagt gctgctgctc ccgcccgagg ctcatgacaa ctcaataaag cactgctttt 180
attttttgca gtcttcaatt tgagaaaggt gagaaataat gttttccaat aaatgagatt 240

cataccatta aaa                                                    253

<210> 37
<211> 1884
<212> DNA
<213> Homo sapiens

<400> 37
tctggtggcc cttccggcca cccctttaac cccagctttc cctccccctt ctttcgatca 60
gagatcggcg gagaccctcg aagtgcgcaa acttgacact caccctgacc ggactggggt 120
tttaaggggt gtggcaggag gttttggact cgatgagttt ccaccgaaat gtcggagaag 180
tcaggccaga gcacaaaagc aaaggatggg aaaaagtatg caacactcag tttatttaat 240
acttacaagg ggaaatcatt agaaacacag aaaaccacag ctcgacatgg attacagagt 300
cttggaaaag tcggtatttc acggcgtatg cctccacctg ctaacctccc aagtcttaaa 360
gcagaaaaca aaggcaatga tcctaatgta aacattgtac ctaaagatgg cacagggtgg 420
gcatcaaaac aagagcaaca tgaagaagaa aaaacaccag aagtgccacc agcacagcca 480
aaacctgggg ttgcagctcc cccagaagta gcacctgctc ccaaatcatg ggccagtaac 540
aagcaaggtg ggcaaggaga tggaatccaa gtgaatagtc agtttcagca agaatttccc 600
agcctgcagg cagctgggga tcaggaaaaa aaagaaaagg aaacaaatga tgacaactat 660
ggacctggac ccagtttacg tccaccaaat gttgcttgtt ggagagatgg tggtaaggct 720
gctggctcac cttcgtcatc tgatcaagat gaaaagctcc ctggccagga tgaaagcaca 780
gctggaacat cagagcaaaa tgatatcctc aaagtggtgg aaaagaggat agcttgtggt 840
cctccacagg ctaaactgaa tggacagcag actgctctcg cttcccagta tagagctatg 900
atgcctcctt atatgttcca acagtatccg aggatgacat atcctcctct acatggtccc 960
atgagattcc caccttcttt atctgaaaca aacaaaggcc ttcgaggaag aggcccacct 1020
ccttcatggg cctctgagcc tgaacgccca tccattctta gtgcatcaga actgaaggag 1080
cttgataaat ttgataacct agatgctgaa gctgatgaag gttgggcagg tgctcagatg 1140
gaagtagatt atacagagca actgaatttc agtgatgatg atgaacaagg aagtaacagt 1200
cctaaagaga ataacagtga ggatcaaggt tcaaaagcct ctgaaaacaa cgaaaacaaa 1260
aaagaaacag atgaagtttc caacactaaa tcatcttccc aaatacctgc ccaaccatca 1320
gtagcaaaag ttccctatgg gaaaggacct tcatttaatc aggaacgtgg aacatcttca 1380
catctgccac cacctccaaa gttgcttgca cagcagcatc cacctccaga tcgacaggca 1440
gtacctggaa gaccaggccc ctttccctcc aagcagcaag tagctgatga agatgaaata 1500
tggaagcaaa gacgaagaca acaatcagaa atttctgcag cagtagaacg tgctcgtaaa 1560
cggcgtgaag aggaagagcg aagaatggaa gaacaaagga aggcagcttg tgcggagaaa 1620

ctgaaacgat tggatgagaa gcttggcatc ctggaaaaac aaccatctcc agaggaaatt 1680
agggaaaggg agcgagaaaa agaacgggag cgtgagaaag aacttgaaaa agaacaagaa 1740
caggagcgag agaaggagag ggaaaaagac agagagagac agcaggaaaa ggagaaagag 1800
ctggagaagg agcaggaaaa acaaagagaa atggagaaag aaagaaagca agaaaaagaa 1860
aaagaactag aacggcagaa aaaa 1884

<210> 38
<211> 1004
<212> DNA
<213> Homo sapiens

<400> 38
cgcaaaatta accccctaat aaaattaatt aaccactcat tcatcgacct ccccacccca 60
tccaacatct ccgcatgatg aaacttcggc tcactccttg gcgcctgcct gatcctccaa 120
atcaccacag gactattcct agccatgcac tactcaccag acgcctcaac cgccttttca 180
tcaatcgccc acatcactcg agacgtaaat tatggctgaa tcatccgcta ccttcacgcc 240
aatggcgcct caatattctt tatctgcctc ttcctacaca tcgggcgagg cctatattac 300
ggatcatttc tctactcaga aacctgaaac atcggcatta tcctcctgct tgcaactata 360
gcaacagcct tcataggcta tgtcctcccg tgaggccaaa tatcattctg aggggccaca 420
gtaattacaa acttactatc cgccatccca tacattggga cagacctagt tcaatgaatc 480
tgaagaggct actcagtaga cagtcccacc ctcacacgat tctttacctt tcacttcatc 540
ttgcccttca ttattgcagc cctagcagca ctccacctcc tattcttgca cgaaacggga 600
tcaaacaacc ccctaggaat cacctcccat tccgataaaa tcaccttcca cccttactac 660
acaatcaaag acgccctcgg cttacttctc ttccttctct ccttaatgac attaacacta 720
ttctcaccag acctcctagg cgacccagac aattataccc tagccaaccc cttaaacacc 780
cctccccaca tcaagcccga atgatatttc ctattcgcct acacaattct ccgatccgtc 840
cctaacaaac taggaggcgt ccttgcccta ttactatcca tcctcatcct agcaataatc 900
cccatcctcc atatatccaa acaacaaagc ataatatttc gcccactaag ccaatcactt 960
tattgactcc tagccgcaga cctcctcatt ctaacctgaa tcgg 1004

<210> 39
<211> 1129
<212> DNA
<213> Homo sapiens

<400> 39
aaagtgtgag agggtccgta gttgggtcaa ctttgactcc tctcgcctgc ccggatcctt 60
aagggcctcc tcgtcctccc ggtctccggt cgctgccggg tctgtgcgcc ggtccgcgcc 120
cgccctcgct ctgccatggg cgcttccagc tcctccgcgc tggcccgcct cggcctccca 180
gcccggccct ggcccaggtg gctcggggtc gccgcgctag gactggccgc cgtggccctg 240
gggactgtcg cctggcgccg cgcatggccc aggcggcgcc ggcggctgca gcaggtgggc 300
accgtggcga agctctggat ctacccggtg aaatcctgca aaggggtgcc ggtgagcgag 360
gctgagtgca cggccatggg gctgcgcagc ggcaacctgc gggacaggtt ttggctggtg 420
attaaggaag atggacacat ggtcactgcc cgacaggagc ctcgcctcgt gctcatctcc 480
atcatttatg agaataactg cctgatcttc agggctccag acatggacca gctggttttg 540
cctagcaagc agccttcctc aaacaaactc cacaactgca ggatatttgg ccttgacatt 600
aaaggcagag actgtggcaa tgaggcagct aagtggttca ccaacttctt gaaaactgaa 660
gcgtatagat tggttcaatt tgagacaaac atgaagggaa gaacatcaag aaaacttctc 720
cccactcttg atcagaattt ccaggtggcc tacccagact actgcccgct cctgatcatg 780
acagatgcct ccctggtaga tttgaatacc aggatggaga agaaaatgaa aatggagaat 840
ttcaggccaa atattgtggt gaccggctgt gatgcttttg aggaggcttc agcaaccagg 900
agggattgac tgagatctta acaacagcag caacgataca tcagcaaatc cttattatcc 960
agccttcaac tatctttacc ctggaaaaca atctcgattt ttgacttttc aaagttgtgt 1020
atgctccagg ttaatgcaag gaaagtatta gaggggggaa tatgaaagta tatatataaa 1080
ttttaggtac tgaaggcttt aaaaataatt aagatcatca aaaatgcta 1129

<210> 40
<211> 821
<212> DNA
<213> Homo sapiens

<400> 40
gtcaactatg ctggctggac tggctgcctt gttcctggcc taggacttag cttcataact 60
atcacctgca ccgactaggc tgaggtgctg gtacttgccc caacccctac ttttgtattt 120
atatgtgtgt gtgtgtgtgc gtgcgtgcgt gcgtgcgtgt atgtttggtc tggaccagct 180
tctgccagcc cctggccttt actttcttcc ttgcctatgc agggcaaaca aaatgtgaaa 240
ttctgccctc agctgagctg agtaagggct cctgggggtt ggctggagat gggtgtggca 300
tctgtccagg cctggaaccg tctcaagaca gtgctggcaa agctgcagta ttgagatgct 360
aaggagctga tgccacctct ttgtcttccc ctaaaggaga acatggggat aacatgggtg 420

tgtgcccaca acactctagg tgcagagccc ctgtggcaaa gtattacagg gtgtgggtgg 480
ggattaccct gaatcgggga ttttaatgat ggaagcaggc agagcctggt gggtgattct 540
gtcaacagaa aattgcaatc atgcaggggc tgggagggtt aggatgaaaa aactggggcc 600
attggaggcc cactgtaggt gggagggagc tgattttggg gtggggggtg ggactagagg 660
gcaatactga aggggttaaa caggtttttg ctcctcaaga atttgtttgc ctgggcccag 720
gattggaggg cttcacacca ataccctgtg tatacaagaa tcagatttat aatacttccc 780
cttttttgtt acgtatgaac actataaacc aaattatttt g 821

<210> 41
<211> 544
<212> DNA
<213> Homo sapiens

<400> 41
tcatttggct cactatgaaa agcctttaat taatctcttc aaacaagtta tttccttaat 60
ccacaagcag tggttacact tgctttgcat tcttgtctgg ttcctaactc tagagccctt 120
ctccctggct tagccagtaa gctgagcccc tggctgcgtt cagccggccc gcctgagaga 180
cactagggga aatagctttt gtgggcaagc agggtggccg gtggtgctca gcagtctttc 240
cagtggctgt gtccctcctc caaatgtgga caggccatga cagagtctta gcccaagtcc 300
cacagatccc caaaagttct gttgattgct tcaggggatc agtgaaaatt agggaatttt 360
gtgtgttgct atatacattt tttctgggga gatgagcttc tcattgagat ctgtgactca 420
gaatcgacta agccaccata agtctggatt tctccccagc tcccaaggcc cttttgggtc 480
cagaagacct gcatatgggc tgttgactca tgcaaatgag gtatctgaac tgcagcttca 540
gtat 544

<210> 42
<211> 1067
<212> DNA
<213> Homo sapiens

<400> 42
cctgtcagtc gccatcatac ctcgagtgag gcccagctag ataatgactt gtccaagatg 60
gcacacgtgg aaagttgatc tgcaccagaa cccggatgac tgtcaccttg aagcgtcctg 120
ttctccttct gtgctgtccc aggaagtgtc tggcgggcgt gggcagcaca gctctacact 180

gtacgattca ctagggcatc ctgcgagcct cactagcctt ctggttcatg cctttgacaa 240
gcatttttgt gccccctctg cttactgtga cagtcgatga tgaatcttgc gttgccattt 300
tctgctgtgg gtaactgcgt gcagtgtctt gccttgcttt ctcttcttac tgtcccacag 360
cttggtttca tgttacaaac agaaaagctc gaggctccca ccccgccaca tcccaacttc 420
atttccccct cactgtagcc catttccacc ccaccacaaa gttgccacag gttttctttg 480
tatagaatat ttattttgaa gctctatttt aatagtattt attttagaaa gtctactatt 540
gtaagagttc ttctgtttgt gaagaaaaaa acaagttaaa aactgaatgt actgatttag 600
aaaatatata taaatatata ttgttaaata tactttgatt gcgccactgc actccagcct 660
tggcgaccag actaagacgc tgtctcaaaa aaaaacaaaa acgacaaaaa aaaaacaaaa 720
cagaaaaaat aaactaaggc aatgacagtc cctggcaaat gctgggaggg aggcagcagt 780
ggtcagggaa ggtaaccctg aagcaggact tgtaaagcaa ataagattgg gaggccaagg 840
tgggtggatc acgaggtcag gagttcgaga ccagcctggc caacatagtg aaaccccgtc 900
tttactaaaa atacaaaaaa attagccagg tgtggtggtg ggtgcctgta gtcccagcta 960
cttgggaggc tgaggcagga gaatctcgaa cccaggaggc ggaggttaca gtcagctgag 1020
accgcaccat tgcactccag cctgggtgac agagcaagat tccgtct 1067

<210> 43
<211> 1560
<212> DNA
<213> Homo sapiens

<400> 43
ttaagggaaa aaatgactgg ctatattcag tcagatatgg caaaaagtct caaggtgtta 60
atgtgaatga ttaaggtctt ggggggggtg tcccctatca gactacaggt gtttagaggc 120
acagaaaaag gtgcagttgg gttcttaatg tgaaatgatg agaagcacaa ctccagtgtg 180
tctctttgtg tagaatgtca gcagacaccc cctgctagat gtgctggatc atgggaaagc 240
atttccattt gttactagat tgttcagaag ttttaattta tgatgggtgt ggtggctcat 300
gcctgtagtc ccagcactgt gggaggctga ggcaggagga tcatctgagg ccaagagttc 360
aagatcagcc tgggcaacat agtgataccc tatctcttaa aaaagaagaa gtttttaaat 420
ttgaaataat aataggtact ggatttatgc aaatgtcttt tctgcgtctt ttgagatgag 480
tatcaggttt ttttttttcc ttttatcatc tgatgatgaa cttaatgttt ccatttgtat 540
taatggaata ctaagtccct ctgtgatttc tgaaccaagc tattcctagg cctgagtttt 600
attttgttga cacagaaata aattagaagg ccaagcgtgg tggcatgtgc ctgtagtcct 660
agttgctgag gtaagaggat tgcttgagcc caggagttca aggctgcagc aagctttgat 720
tgcgccactg cactccagcc ttggcgacag actaagacgc tgtctcaaaa aaaaacaaaa 780

acgacaaaaa aaaaacaaaa cagaaaaaat aaactaaggc aatgacagtc cctggcaaat 840
gctgggaggg aggcagcagt ggtcagggaa ggtaaccctg aagcaggact tgtaaagcaa 900
ataagattgg gaggccaagg tgggtggatc acgaggtcag gagttcgaga ccagcctggc 960
caacatagtg aaaccccgtc tttactaaaa atacaaaaaa attagccagg tgtggtggtg 1020
ggtgcctgta gtcccagcta cttgggaggc tgaggcagga gaatctcgaa cccaggaggc 1080
ggaggttaca gtcagctgag accgcaccat tgcactccag cctgggtgac agagcaagat 1140
tccgtctcaa aaaaaaaaaa aaaaaaaaaa ccaagaagaa aaggaatgaa ttagaacttc 1200
ttctgcttgg acttaagggc atcatcaggc aggttttggg taggatagca ggggaggcag 1260
agacatagtc ggggtcagtg gtcatgagtg tggctttgag cccaaaaact tggtttctgt 1320
tccctacttt gccactcagt agtgcatgac tttggccaaa tttcttaaat tcatgaagca 1380
agtttccggg tgaatgaaat ggggataaaa atagtgttca aacctatccg ttggtttgtg 1440
tgaaactgaa atgaatagta tcgtgcaggt acttgtgagc aaggggagct gctgtttcct 1500
gtccctttat gatgggaaat atctagacaa gttcccaacc ctctgcactg caggctgcat 1560

<210> 44
<211> 397
<212> DNA
<213> Homo sapiens

<400> 44
agagtcacta tccggaaatc taagaatatc ctctttgtca tcacaaaacc agatgtctac 60
aagagccctg cttcagatac ttacatagtt tttggggaag ccaagatcga agatttatcc 120
cagcaagcac aactagcagc tgctgagaaa ttcaaagttc aaggtgaagc tgtctcaaac 180
attcaagaaa acacacagac tccaactgta caagaggaga gtgaagagga agaggtcgat 240
gaaacaggtg tagaagttaa ggacatagaa ttggtcatgt cacaagcaaa tgtgtcgaga 300
gcaaaggcag tccgagccct gaagaacaac agtaatgata ttgtaaatgc gattatggaa 360
ttaacaatgt aaccatatgg aagcaacttt ttttggt 397

<210> 45
<211> 931
<212> DNA
<213> Homo sapiens

<400> 45

gcctagccat gtgatttcac ttccactcca taacgctcct catactaggc ctactaacca 60
acacactaac catataccaa tgatggcgcg atgtaacacg agaaagcaca taccaaggcc 120
accacacacc acctgtccaa aaaggccttc gatacgggat aatcctattt attacctcag 180
aagttttttt cttcgcagga tttttctgag ccttttacca ctccagccta gcccctaccc 240
cccaattagg agggcactgg cccccaacag gcatcacccc gctaaatccc ctagaagtcc 300
cactcctaaa cacatccgta ttactcgcat caggagtatc aatcacctga gctcaccata 360
gtctaataga aaacaaccga aaccaaataa ttcaagcact gcttattaca attttactgg 420
gtctctattt taccctccta caagcctcag agtacttcga gtctcccttc accatttccg 480
acggcatcta cggctcaaca ttttttgtag ccacaggctt ccacggactt cacgtcatta 540
ttggctcaac tttcctcact atctgcttca tccgccaact aatatttcac tttacatcca 600
aacatcactt tggcttcgaa gccgccgcct gatactggca ttttgtagat gtggtttgac 660
tatttctgta tgtctccatc tattgatgag ggtcttactc ttttagtata aatagtaccg 720
ttaacttcca attaactagt tttgacaaca ttcaaaaaag agtaataaac ttcgccttaa 780
ttttaataat caacaccctc ctagccttac tactaataat tattacattt tgactaccac 840
aactcaacgg ctacatagaa aaatccaccc cttacgagtg cggcttcgac cctatatccc 900
ccgcccgcgt ccctttctcc ataaaattct t 931

<210> 46
<211> 968
<212> DNA
<213> Homo sapiens

<400> 46
gagcacatgg ttattactga tcgcattgaa aacattgatc acctgggttt ctttatttat 60
cgactgtgtc atgacaagga aacttacaaa ctgcaacgca gagaaactat taaaggtatt 120
cagaaacgtg aagccagcaa ttgtttcgca attcggcatt ttgaaaacaa atttgccgtg 180
gaaactttaa tttgttcttg aacagtcaag aaaaacatta ttgaggaaaa ttaatatcac 240
agcataaccc caccctttac attttgtgca gtgattattt tttaaagtct tctttcatgt 300
aagtagcaaa cagggcttta ctatcttttc atctcattaa ttcaattaaa accattacct 360
taaaattttt ttctttcgaa gtgtggtgtc ttttatattt gaattagtaa ctgtatgaag 420
tcatagataa tagtacatgt caccttaggt agtaggaaga attacaattt ctttaaatca 480
tttatctgga tttttatgtt ttattagcat tttcaagaag acggattatc tagagaataa 540
tcatatatat gcatacgtaa aaatggacca cagtgactta tttgtagttg ttagttgccc 600
tgctacctag tttgttagtg catttgagca cacattttaa ttttcctcta attaaaatgt 660
gcagtatttt cagtgtcaaa tatatttaac tatttagaga atgatttcca cctttatgtt 720

ttaatatcct aggcatctgc tgtaataata ttttagaaaa tgtttggaat ttaagaaata 780
acttgtgtta ctaatttgta taacccatat ctgtgcaatg gaatataaat atcacaaagt 840
tgtttaacta gactgcgtgt tgtttttccc gtataataaa accaaagaat agtttggttc 900
ttcaaatctt aagagaatcc acataaaaga agaaactatt ttttaaaaat tcacttctat 960
atatacaa 968

<210> 47
<211> 904
<212> DNA
<213> Homo sapiens

<400> 47
ctgggggaga agagcctgcc gaggaggact ccgaggactg gtgcgtgccc tgcagcgacg 60
aggaggtgga gctgcctgcg gatgggcagc cctggatgcc cccgccctcc gaaatccagc 120
ggctctatga actgctggct gcccacggta ctctggagct gcaagccgag atcctgcccc 180
gccggcctcc cacgccggag gcccagagcg aagaggagag atccgatgag gagccggagg 240
ccaaagaaga ggaagaggaa aaaccacaca tgcccacgga atttgatttt gatgatgagc 300
cagtgacacc aaaggactcc ctgattgacc ggagacgcac cccaggaagc tcagcccgga 360
gccagaaacg ggaggcccgc ctggacaagg tgctgtcgga catgaagaga cacaagaagc 420
tggaggagca gatccttcgt accgggaggg acctcttcag cctggactcg gaggacccca 480
gccccgccag ccccccactc cgatcctccg ggagtagtct cttccctcgg cagcggaaat 540
actgattccc actgctcctg cctctagggt gcagtgtccg tacctgctgg agcctgggcc 600
ctccttcccc agcccagaca ttgagaaact tgggaagaag agagaaacct caagctccca 660
aacagcacgt tgcgggaaag aggaagagag agtgtgagtg tgtgtgtgtg ttttttctat 720
tgaacacctg tagagtgtgt gtgtgtgttt tctattgaac acctatagag agagtgtgtg 780
tgttttctat tgaacatcta tatagagaga gtgtgtgagt gtgtgttttc tattgaacac 840
ctattcagag acctggactg aattttctga gtctgaaata aaagatgcag agctatcatc 900
tctt 904

<210> 48
<211> 877
<212> DNA
<213> Homo sapiens

<400> 48
aaaggacagt ctcttcaata aatggtgttg ggaaaactgg atatccatat gcaaaagaat 60
gaaattggac ctttatctca caccattagc aaaaatcagc tcaaaatgaa ttaaagactt 120
aaacataaga cctgaaattg taaaactact ggaagaaaac gggaaaagtt ctaccacatt 180
tgtctgcaaa atgtcatata tattcaacag aatactattc agccctagaa aagaaggaaa 240
tcctgtcatt tgtgacaata tgaatgaacc tggaagacat tatattaagc aaaataagcc 300
aggcatggaa agacaatatt gcatgatctc acttatatgt ggaatctaaa aaaaattgga 360
cttacaaaag aaaaaaagag aatggtggtt actagatgcc ggggatggga atgcggatgg 420
ggaatgcaga gatgttgatc aaaaggtaca aagtttcagc tgggtgcagt ggctcacgcc 480
tgtaatccca gcactttggg aggccaaggc aggcagatca cttgaggtca ggagtttgag 540
accagcctgg ccaacatggt gaaaccctgt ccttgctaaa ataaaaaaat tagcccagtg 600
tggtggcatg cacctgtaat cccagctact caggaggtcg aggcggcaga attgcttgaa 660
cccaggaggt ggaggttgta gtgagctgag attgtgccac tgcactccag cctgggtgac 720
agagcaagac tctgtctcaa aaaaaaaaaa gtacgaagtt tcagttagac aggaaaagtg 780
agttttcagg acctattgaa cagcatggtg accatagtta ataataatat atatttcaga 840
ttgctaaaag agcttatttt aagtgttctt actacaa 877

<210> 49
<211> 793
<212> DNA
<213> Homo sapiens

<400> 49
acgttgtagg cccctacggg ctactacaac ccttcgctga cgccataaaa ctcttcacca 60
aagagcccct aaaacccgcc acatctacca tcaccctcta catcaccgcc ccgaccttag 120
ctctcaccat cgctcttcta ctatgaaccc ccctccccat acccaacccc ctggtcaacc 180
tcaacctagg cctcctattt attctagcca cctctagcct agccgtttac tcaatcctct 240
gatcagggtg agcatcaaac tcaaactacg ccctgatcgg cgcactgcga gcagtagccc 300
aaacaatctc atatgaagtc accctagcca tcattctact atcaacatta ctaataagtg 360
gctcctttaa cctctccacc cttatcacaa cacaagaaca cctctgatta ctcctgccat 420
catgaccctt ggccataata tgatttatct ccacactagc agagaccaac cgaaccccct 480
tcgaccttgc cgaaggggag tccgaactag tctcaggctt caacatcgaa tacgccgcag 540
gccccttcgc cctattcttc atagccgaat acacaaacat tattataata aacaccctca 600
ccactacaat cttcctagga acaacatatg acgcactctc ccctgaactc tacacaacat 660
attttgtcac caagacccta cttctaacct ccctgttctt atgaattcga acagcatacc 720

cccgattccg ctacgaccaa ctcatacacc tcctatgaaa aaacttccta ccactcaccc 780
tagcattact tat 793

<210> 50
<211> 2086
<212> DNA
<213> Homo sapiens

<400> 50
tattttcagt agagacgggg tttcaccatg ttagccagga tggtctcgat ctcctgacct 60
cgtgatccgc ccgcctcggc ctcccaaagt gctgggatta caggcatgag ccaccgggcc 120
cggatgtctt ggtttttctt gatatttgaa acagtaggtc aaaattggac atgtctagta 180
ggcggttgct ggtttagtaa taaagggtag tagctgtaat tgcccagttt gaatagtgtt 240
catgttttta aggatttcac aaacaaatct tcatcgaata aatgttttta aaaaatgatt 300
actgattaga ttcaatataa attaaggaag gtcccatcat atctcttgca caaatcattg 360
ccttccagaa ttaagcacag catcgatatg tatgtcttac atgacatatt gctctgcaat 420
gtgatggaat caacagaaca agatgtttgg agtcagatgg ctaaactagg atttaaagtt 480
cacattattt actagttaac tgtggatgct aaggcaatat tcttgaagtc tccatgtctt 540
gatttcctct tagtgaaatg ggaatcataa taataataat tttacagggt tggtgtaaaa 600
acgaaatgac tgcctgcact caatacaata cctattatgg ttatgttagt tatattatga 660
ttaatgtatt agtatactat tgataaaatg aatatgaaat tgctagtagc cagctcacaa 720
tattagtatg acaaaaatta tttcaatttg gtttaaatac aatacctgtg gtcaataaca 780
tcatcttttc ttaaaactta atgcactgag cactgtcaat tacaatgaaa aaaaaatgca 840
agtaacccag ttggactatc cattaactga gaaaatataa tatgaaaaag ccaccatgaa 900
ttcaggacca cttatgaatg tagctgtatt ttattgatag aatttatatg tgcttagaaa 960
aaacatagta cattttatgt tagatatatt tattcagatt accaaagaaa atgcttactg 1020
tacatatgga ttgatgggcc ctatccaata actttcatca ctcctcaagg gcccttggca 1080
cacaggctgg gaatcactga tgttggttaa ggtcacaata gacaggggag cctcatgctt 1140
ataattctgc ttgccaagtg gaaatgctaa cctgctcttt cccaggattt tagaaaaaca 1200
tttgaaatat ttcacaagat aaatgaaaat gggtaaacac aaggccttaa ggaaaatata 1260
cttctggtgc atttattaag cacttggatc tgatgcatga cacctgctta aaactcaaag 1320
aattatcaca ggctttgctg aggaagaaag aacaagaatt atgactgcac acaggctcat 1380
acttcagcaa gtcaatgttt ttaaatcaat gaaaaacaat ccagtgattg tgtttgctac 1440
aaagggaaag caatgtaaga tgacatacag tatttcaagc tgttcaaatt actgatggca 1500
cagtggcaag atatatatcc attaaaacca aatagttgtt tcaaagtatg ttggccaaca 1560

gagataaata atgtttctct gaagtgaata taaaataatg tctctcttca gaatgtagga 1620
ccctataatg gctttagcca caaatacctc ttgcttattt ggggaatacg cattttccac 1680
cttcattcag aaattcttta gttagctcaa caaaattcat ctttcacctc aacaaaaaag 1740
catgcgcatt aagtatcaaa gtatgtatat ttctacagta ggctcactga tggtctcatt 1800
cctattggta aatttgtcat agtgaatgag taaagtatga ataaatacaa tgtgttgaat 1860
gtagaggcta ctttcacaag aatattgcat catcagtggg ccaatgttaa gagcatgttt 1920
gtgaggaaga ggaacataca atttctcttt ttttcttaat tcttctctta tcagcatttt 1980
aattaataaa cataaaaagg taacactgat acacaaagta ttaaatcacc atccattcgt 2040
ctttaaagat ttgcattctc ctaatcagtg aaaacaattc tttctt 2086

<210> 51
<211> 941
<212> DNA
<213> Homo sapiens

<400> 51
aaataaacat gctagctttt attccagttc taaccaaaaa aataaaccct cgttccacag 60
aagctgccat caagtatttc ctcacgcaag caaccgcatc cataatcctt ctaatagcta 120
tcctcttcaa caatatactc tccggacaat gaaccataac caatactacc aatcaatact 180
catcattaat aatcataatg gctatagcaa taaaactagg aatagccccc tttcacttct 240
gagtcccaga ggttacccaa ggcacccctc tgacatccgg cctgcttctt ctcacatgac 300
aaaaactagc ccccatctca atcatatacc aaatctctcc ctcactaaac gtaagccttc 360
tcctcactct ctcaatctta tccatcatag caggcagttg aggtggatta aaccaaaccc 420
agctacgcaa aatcttagca tactcctcaa ttacccatat aggatgaata atagcagttc 480
taccgtacaa ccctaacata accattctta atttaactat ttatattatc ctaactacta 540
ccgcattcct actactcaac ttaaactcca gcaccacgac cctactacta tctcgcacct 600
gaaacaagct aacatgacta acacccttaa ttccatccac cctcctctcc ctaggaggcc 660
tgcccccgct aaccggcttt ttgcccaaat gggccattat cgaagaattc acaaaaaaca 720
atagcctcat catccccacc atcatagcca ccatcaccct ccttaacctc tacttctacc 780
tacgcctaat ctactccacc tcaatcacac tactccccat atctaacaac gtaaaaataa 840
aatgacagtt tgaacataca aaacccaccc cattcctccc cacactcatc gcccttacca 900
cgctactcct acctatctcc ccttttatac taataatctt a 941

<210> 52

<211> 517
<212> DNA
<213> Homo sapiens

<400> 52
ttaaaaatgc cctagcccac ttcttaccac aaggcacacc tacacccctt atccccatac 60
tagttattat cgaaaccatc agcctactca ttcaaccaat agccctggcc gtacgcctaa 120
ccgctaacat tactgcaggc cacctactca tgcacctaat tggaagcgcc accctagcaa 180
tatcaaccat taaccttccc tctacactta tcatcttcac aattctaatt ctactgacta 240
tcctagaaat cgctgtcgcc ttaatccaag cctacgtttt cacacttcta gtaagcctct 300
acctgcacga caacacataa tgacccacca atcacatgcc tatcatatag taaaacccag 360
cccatgaccc ctaacagggg ccctctcagc cctcctaatg acctccggcc tagccatgtg 420
atttcacttc cactccataa cgctcctcat actaggccta ctaaccaaca cactaaccat 480
ataccaatga tggcgcgatg taacacgaga aagcaca 517

<210> 53
<211> 793
<212> DNA
<213> Homo sapiens

<400> 53
ggcgaggcct atattacgga tcatttctct actcagaaac ctgaaacatc ggcattatcc 60
tcctgcttgc aactatagca acagccttca taggctatgt cctcccgtga ggccaaatat 120
cattctgagg ggccacagta attacaaact tactatccgc catcccatac attgggacag 180
acctagttca atgaatctga ggaggctact cagtagacag tcccaccctc acacgattct 240
ttacctttca cttcatcttg cccttcatta ttgcagccct agcagcactc cacctcctat 300
tcttgcacga aacgggatca aacaaccccc taggaatcac ctcccattcc gataaaatca 360
ccttccaccc ttactacaca atcaaagacg ccctcggctt acttctcttc cttctctcct 420
taatgacatt aacactattc tcaccagacc tcctaggcga cccagacaat tataccctag 480
ccaacccctt aaacacccct ccccacatca agcccgaatg atatttccta ttcgcctaca 540
caattctccg atccgtccct aacaaactag gaggcgtcct tgccctatta ctatccatcc 600
tcatcctagc aataatcccc atcctccata tatccaaaca acaaagcata atatttcgcc 660
cactaagcca atcactttat tgactcctag ccgcagacct cctcattcta acctgaatcg 720
gaggacaacc agtaagctac ccttttacca tcattggaca agtagcatcc gtactatact 780
tcacaacaat cct 793

<210> 54
<211> 827
<212> DNA
<213> Homo sapiens

<400> 54
ggaaccttct ggttcctgcg ccaatcgcca ccagtatcaa ttgtgtgagc ttgggtgcga 60
gtgcacgcgt gcgtgagtac ggagagtata tatagatctc tatctcttag caaaggtgaa 120
tgccagatgt aaatggcgcc tctgggcaaa ggaggcttgt attttgcaca ttttataaaa 180
acttgagaga atgagatttc tgcttgtata tttctaaaaa gaggaaggag cccaaaccat 240
cctctcctta ccactcccat ccctgtgagc cctaccttac ccctctgccc ctagccaagg 300
agtgtgaatt tatagatcta actttcatag gcaaaacaaa agcttcgagc tgttgcgtgt 360
gtgagtctgt tgtgtggatg tgcgtgtgtg gtccccagcc ccagactgga ttggaaaagt 420
gcatggtggg ggcctcgggg ctgtccccac gctgtccctt tgccacaagt ctgtggggca 480
agaggctgca atattccgtc ctgggtgtct gggctgctaa cctggcctgc tcaggcttcc 540
caccctgtgc ggggcacacc cccaggaagg gaccctggac acggctccca cgtccaggct 600
taaggtggat gcacttcccg cacctccagt cttctgtgta gcagctttaa cccacgtttg 660
tctgtcacgt ccagtcccga gacggctgag tgaccccaag aaaggcttcc ccgacaccca 720
gacagaggct gcagggctgg ggctgggtga gggtggcggg cctgcgggga cattctactg 780
tgctaaaaag ccactgcaga catagcaata aaaacatgtc attttcc 827

<210> 55
<211> 1562
<212> DNA
<213> Homo sapiens

<400> 55
cccaccgact ccactatgtt gaagaaattc gacaagaagg atgaggagtc aggtggaggc 60
tccaacccat tccagcacct tgagaagagt gcggtactcc aggaggcccg tgtatttaat 120
gaaactccca tcaaccctcg gaaatgtgcc cacatcctca ccaagattct ttatctcata 180
aaccaggggg agcacctggg gaccacggaa gcgaccgagg ccttctttgc catgaccaag 240
ctctttcagt ccaatgatcc cacactccgt cggatgtgct acttgaccat caaggagatg 300
tcttgcattg cagaggatgt catcattgtc accagcagcc taacaaaaga catgactggg 360

aaagaagaca actaccgggg cccggccgtg cgagccctct gccagatcac tgatagcacc 420
atgctgcagg ctattgagcg ctacatgaaa caagccattg tggacaaggt gcccagtgtc 480
tccagctctg ccctcgtgtc ttccttgcac ctgctgaagt gcagctttga cgtggtcaag 540
cgctgggtga atgaggctca ggaggcagca tccagtgata acatcatggt ccagtaccac 600
gcactagggc tcctgtacca tgtgcgtaag aatgaccgcc tagccgtcaa taagatgatc 660
agcaaggtca cacggcatgg ccttaagtct ccctttgcct actgcatgat gatccgggtg 720
gccagcaagc agctggaaga ggaggatggc agccgtgaca gcccactgtt tgacttcatc 780
gagagctgct tgcgcaacaa gcacgagatg gtggtgtatg aagccgcctc ggccatcgtc 840
aatctgccag gctgcagtgc caaagagctg gccccggctg tgtcagtgct ccagcttttc 900
tgcagctcac ccaaggctgc tctccgctat gctgctgttc gtaccctcaa taaggttgcc 960
atgaagcatc cgtcagctgt gacagcttgt aatctggatc tggagaacct ggtcacagat 1020
tcaaaccgca gcattgccac gctggccatc accaccctcc ttaagacggg cagcgagagc 1080
agcatcgacc gcctcatgaa gcagatctcc tccttcatgt cagaaatctc ggatgaattc 1140
aaggtggtgg ttgtccaggc catcagtgcc ctgtgtcaga aatatcctcg caaacacgcc 1200
gtccttatga acttcctgtt caccatgctg cgggaagagg gtggctttga gtataagcgc 1260
gctatcgtgg actgcatcat cagcatcatt gaagagaact cagagagcaa ggagacaggg 1320
ctgtcacatc tgtgcgagtt catcgaggac tgcgagttca cagtgctggc cacccgtatt 1380
ctacatctcc tgggccagga ggggcccaag accaccaatc cctcaaagta catccgcttc 1440
atctataacc gagtggtctt ggagcatgag gaggtccggg caggtgctgt gagtgctctg 1500
gcgaagtttg gagcccagaa tgaagagatg ttacccagta tcttggtgtt gctgaagagg 1560
tg 1562

<210> 56
<211> 678
<212> DNA
<213> Homo sapiens

<400> 56
ccccaaaccc actccacctt actaccagac aaccttagcc aaaccattta cccaaataaa 60
gtataggcga tagaaattga aacctggcgc aatagatata gtaccgcaag ggaaagatga 120
aaaattataa ccaagcataa tatagcaagg actaacccct ataccttctg cataatgaat 180
taactagaaa taactttgca aggagagcca aagctaagac ccccgaaacc agacgagcta 240
cctaagaaca gctaaaagag cacacccgtc tatgtagcaa aatagtggga agatttatag 300
gtagaggcga caaacctacc gagcctggtg atagctggtt gtccaagata gaatcttagt 360
tcaactttaa atttgcccac agaaccctct aaatcccctt gtaaatttaa ctgttagtcc 420

aaagaggaac agctctttgg acactaggaa aaaaccttgt agagagagta aaaaatttaa 480
cacccatagt aggcctaaaa gcagccacca attaagaaag cgttcaagct caacacccac 540
tacctaaaaa atcccaaaca tataactgaa ctcctcacac ccaattggac caatctatca 600
ccctatagaa gaactaatgt tagtataagt aacatgaaaa cattctcctc cgcataagcc 660
tgcgtcagat taaaacac 678

<210> 57
<211> 1338
<212> DNA
<213> Homo sapiens

<400> 57
tcacaacaag caaacctgga gtatccttgg tctactccat gccctcccgg aacctgtccc 60
tgcggctgga gggtctccag gagaaagact ctggccccta cagctgctcc gtgaatgtgc 120
aagacaaaca aggcaaatct aggggccaca gcatcaaaac cttagaactc aatgtactgg 180
ttcctccagc tcctccatcc tgccgtctcc agggtgtgcc ccatgtgggg gcaaacgtga 240
ccctgagctg ccagtctcca aggagtaagc ccgctgtcca ataccagtgg gatcggcagc 300
ttccatcctt ccagactttc tttgcaccag cattagatgt catccgtggg tctttaagcc 360
tcaccaacct ttcgtcttcc atggctggag tctatgtctg caaggcccac aatgaggtgg 420
gcactgccca atgtaatgtg acgctggaag tgagcacagg gcctggagct gcagtggttg 480
ctggagctgt tgtgggtacc ctggttggac tggggttgct ggctgggctg gtcctcttgt 540
accaccgccg gggcaaggcc ctggaggagc cagccaatga tatcaaggag gatgccattg 600
ctccccgaac cctgccctgg cccaagagct cagacacaat ctccaagaat gggacccttt 660
cctctgtcac ctccgcacga gccctccggc caccccatgg ccctcccagg cctggtgcat 720
tgacccccac gcccagtctc tccagccagg ccctgccctc accaagactg cccacgacag 780
atggggccca ccctcaacca atatccccca tccctggtgg ggtttcttcc tctggcttga 840
gccgcatggg tgctgtgcct gtgatgatgc ctgcccagag tcaagctggc tctctggtat 900
gatgacccca ccactcattg gctaaaggat ttggggtctc tccttcctat aagggtcacc 960
tctagcacag aggcctgagt catgggaaag agtcacactc ctgaccctta gtactctgcc 1020
cccacctctc tttactgtgg gaaaaccatc tcagtaagac ctaagtgtcc aggagacaga 1080
aggagaagag gaagtggatc tggaattggg aggagcctcc acccacccct gactcctcct 1140
tatgaagcca gctgctgaaa ttagctactc accaagagtg aggggcagag acttccagtc 1200
actgagtctc ccaggccccc ttgatctgta ccccacccct atctaacacc acccttggct 1260
cccactccag ctccctgtat tgatataacc tgtcaggctg gcttggttag gttttactgg 1320
ggcagaggat agggaatc 1338

<210> 58
<211> 1999
<212> DNA
<213> Homo sapiens

<400> 58
cccatttttg tatacctatc atccttccaa taaatcactc tttgcttagt ttaaccagac 60
tcagtttctg ttgctctcaa ctaaaacagt taacagataa tgattctata ttaaaatctc 120
ttagaatttc ttttctatat ttttattggg tatttatcat gttagcagag cctgtaaact 180
ttaagggctt caagaaattt ttaaaaaata tttcccggcc agatgcggtg gctcacacct 240
gtaatactag cactttggga ggccaaggcg ggcggatcac ctgaggtcag gagtttgaga 300
ccagcctgac caacatggag aaaccccatt tctactaaaa atacaaaatt agccgggcgt 360
ggtggtgcat gcctgtaatc ccagctactc gggaggctga ggcaggagaa ttgcttgaac 420
ctgggaggcg gaggttgcgg tgagtcaaga tcctgcaacc acattccggc ctgggcaaaa 480
aagcaagact ctttctcaaa aaaaaaaaaa aaaaaaaggt aaaatgacaa agtcatgcac 540
acacatgcta atctggaaag ctataaatca aactgaaaac actggttatc ttgtagtgga 600
atggctggcc actttgactt tctactttaa acatttggcc tatttattta ttttaaagag 660
acagggtctc actctgttgc tgcccaagct ggagtagagt ggcgtaacca tagctcactg 720
taacctcaaa ctcctgggct catgtaatcc tctcacttca gcctccagag tagctgagac 780
tatacaagtg cccgccacca tacctggtta attttttttt ttttttagat gaggtttccc 840
tatgttgccc aggctggtct tgaactcctg gcctcaagga atcctcccag tttggccttc 900
caaagtgctg gaattgcagg cataagccac catgcctggc ccagaacaaa cttattctta 960
tgtcatgttt gtggcaacaa aaattcaaaa aataatgaga cttccaggtt agctttaata 1020
gagagaggca agctagtgct ttcacagtaa caactagata aaactaaata gattattcaa 1080
ataaataaat ggctttaaag attgaagagc tgtcttctat gcatagcaaa gaggactagc 1140
agaattaaaa ttccagaggg agaagaggcc tctctaggtg agctgaaagt gccagccatt 1200
tcctctctct acttgtggta cttgtagagc tttagcgtga gtgatgataa ctggggcttg 1260
ccataggcag aactccatca agagaaagaa aaaccagcag aacttatgac agctatatgg 1320
gttggcatga cagactaaat ttcataacag ccctaaattt atggccagtt ttctacacag 1380
gacattcacc tccaggctgg aggcctgaac tagaaaacca aagtgaaatt aagcacaatc 1440
tcacagtgct tagcagataa agctagaggg agtggcctgt aacaaacata caacggatcc 1500
cactcttggg atactacacc gaactgaagg acttaaatct tcagcccagt ccaactcagt 1560
tgtttggata gaggtgtttt atacctcatt gtatctgcct aagactgcaa aatgcaaaca 1620
ttaactttag actctaaagg tctttacaca cattgtctag aaatcgtcaa aaacttctgc 1680

tgcctggcgc agtggctcac gtctgtagtt ccagcatttt gggaggccga ggcaggctga 1740
tcatgaggtc aggacttcga gaccagcctg accaacatgg tgaaaccctc tctctactga 1800
aaatacaaaa atttgccagg catggtggcg cacgcccata atcccagcta ctggggaggc 1860
tgaggcagga gagccgcttg aacccaggag gcaaaggttg cagtgagcca aaaccgcgtg 1920
ccactgcact ccagcctggg caacagagcg agactctgtc tcaaaaaaca acaacaactt 1980
ttgctttccc aggagggag 1999

<210> 59
<211> 957
<212> DNA
<213> Homo sapiens

<400> 59
ggcctgctca aggtggtgtt cgtggtcttc gcctccttgt gtgcctggta ttcggggtac 60
ctgctcgcag agctcattcc agatgcaccc ctgtccagtg ctgcctatag catccgcagc 120
atcggggaga ggcctgtcct caaaggtgag tgccgtgctt ggggcagacg gctgccttca 180
tggcttgtgt gcagagggag gagtggtggt ttctgtccca gctggaggct ggggggccct 240
gacggcttta tatcaggaag gaggaggaga gaagcatttt gcagttactc atttaggtcc 300
cacgccattc caaggcacgt gccgctgctg accccatgtg agactagaac agtgtggctt 360
ctggttacca gcacaaacct gcagccagac cttgaactgt gctctttccg cccagatctt 420
ggcttcctcc cctgggaagc acaggagggt ccaggttctg agatcccgaa tcattttaag 480
gtttctgtcg gactgaagag ctgttggaaa atcttctgta aagtcttggg aagcaaaagc 540
tagtaattga gtagatgtga aagaaagggc agacattgta tgtcggtgaa cacacggcct 600
caaccatcag cttaggaaag gcccagctct ctgtagggcg gctgctttct gtccttcccc 660
acaatcaagg tgggttttct gagagcctgc tgtgtgctgg gcatgtccag gagctaagga 720
gagacggagg acaagcccac gaggtccctg cccctttggc actgataccc tagtgagggg 780
aggtgacagc ttaacgcatg aacaaatgag attcgagaga ggatgggtgg ggaaagcctc 840
tgagaaggag gtgacaattg agaagcatct cagtgaagca agggagtggg ccatgctggg 900
ttctgagggc tgtgtgccag tttgggagca tcatgactta gtctacccag attactg 957

<210> 60
<211> 941
<212> DNA
<213> Homo sapiens

<400> 60
cttaggaaac tggagaggat attcatggtc ttaagcacta gattatagag tttaggttgc 60
cagagtaaca gtttcaaaga tcctggcaca cacagttgct ctttagtaaa tagttcttaa 120
ataaattgag acatgtacat ttattctcaa tttcctcttt cttatttgtt ttccagagtg 180
tttccttact ctcagaagtt taaaaaatta agtacttggg gctgggcgca gtgactcacg 240
cctgtaatcc cagcactttg ggaggctgag gtggccagat cataaggtca agagatcgag 300
atcatcctgg ccaacatggt gaaaccccgt ctctactaaa aatacaaaaa ctagctgagc 360
gtggtggcag gcacctgtag tcccagctat ttgggaggct gaggcagcag aatcacttga 420
acccaggagg ggaggttgca gtgagccgag atcgcgccat gcacaccagc ctgggtgata 480
gagcgagact ccatctccaa aaaaaaaaaa agtacttggg tacagtggct cacacctgta 540
atcctagcat ttggggagac caaggtggga ggatcacttg agcctgggag gtcaaggctg 600
cagtgagaca tgtttgagca ctgcattcca acctgggtga cagagagcct atcttattgt 660
tgtataaagg tttgaaatag gtacaaggtt tggtcattca gtttcaaatt catttctcaa 720
atatccttgt atctcactaa catcatctgt ctaccagtaa catttccaaa tctaatagta 780
cttggcaaga gatggggagg gggttaaatt gcacaattta aaattttcat aattgttatg 840
catcacagtt attgtgatca cgtgataatg ttatgcatgt cttttgttgt gtgtcagccc 900
caacttttgt ctcagcattt gaaccatgca tacatcttat c 941

<210> 61
<211> 2131
<212> DNA
<213> Homo sapiens

<400> 61
   agaagctgcc atgacctgaa ttaggtaatg ggtagtggac agaagggcac aactgcccat 60
   gcctgggagg gaacaaaggg gtgttgactc ctgtgaaccc acttcccaag ctctatatca 120
   gagctcctgg gctgcatagg agtggccagg ctaacaacct gaccccttcc cttcctccct 180
   gggcagctcc ctgcggtgtg gccccccaag cacgcatcac aggtggcagc agtgcagtcg 240
   ccggtcagtg gccctggcag gtcagcatca cctatgaagg cgtccatgtg tgtggtggct 300
   ctctcgtgtc tgagcagtgg gtgctgtcag ctgctcactg cttccccagg taccaaatgg 360
   tgaaagtcaa aagtgggctg gaggtcaaag ttaatgggtc aatccaggtc agaggttggg 420
   gttcttgggt tgggctgaga gtgtcagttc taagtctgga aggaccagtt aggagtgaag 480
   gtgaggggtc agaggttaca tgataaaact aaggtgcaat ttgagaccta aaaggggctg 540
   atcggaatca ggggttggca gtgggggttg ggaacaaggg agaggtctcc tgggtctcag 600
   cctttgcccc ctgcctgcag cgagcaccac aaggaagcct atgaggtcaa gctgggggcc 660
   caccagctag actcctactc cgaggacgcc aaggtcagca ccctgaagga catcatcccc 720
   caccccagct acctccagga gggctcccag ggcgacattg cactcctcca actcagcaga 780
   cccatcacct tctcccgcta catccggccc atctgcctcc ctgcagccaa cgcctccttc 840
   cccaacggcc tccactgcac tgtcactggc tggggtcatg tggccccctc aggtgaggtg 900
   ggacgtgggt gcctagaggt gtggaggggc acctgacttg gggaggggcc cagggtaagc 960
   ctcttttacc cccacagtga gcctcctgac gcccaagcca ctgcagcaac tcgaggtgcc 1020
   tctgatcagt cgtgagacgt gtaactgcct gtacaacatc gacgccaagc ctgaggagcc 1080
   gcactttgtc caagaggaca tggtgtgtgc tggctatgtg gaggggggca aggacgcctg 1140
   ccaggtaagc acaggcccgg gggcagatga ccagtgcaac ttcggaaagg aggcctggcc 1200
   cggtcctgat ggctgctgtg tggcttctct cctcagggtg actctggggg cccactctcc 1260
   tgccctgtgg agggtctctg gtacctgacg ggcattgtga gctggggaga tgcctgtggg 1320
   gcccgcaaca ggcctggtgt gtacactctg gcctccagct atgcctcctg gatccaaagc 1380
   aaggtgacag aactccagcc tcgtgtggtg ccccaaaccc aggagtccca gcccgacagc 1440
   aacctctgtg gcagccacct ggccttcagc tctgccccag cccagggctt gctgaggccc 1500
   atccttttcc tgcctctggg cctggctctg ggcctcctct ccccatggct cagcgagcac 1560
   tgagctggcc ctacttccag gatggatgca tcacactcaa ggacaggagc ctggtccttc 1620
   cctgatggcc tttggaccca gggcctgact tgagccactc cttccttcag gactctgcgg 1680
   gaggctgggg ccccatcttg atctttgagc ccattcttct gggtgtgctt tttgggacca 1740
   tcactgagag tcaggagttt tactgcctgt agcaatggcc agagcctctg gcccctcacc 1800
   caccatggac cagcccattg gccgagctcc tggggagctc ctgggaccct tggctatgaa 1860
   aatgagccct ggctcccacc tgtttctgga agactgctcc cggcccgctg cccagactga 1920
   tgagcacatc tctctgccct ctccctgtgt tctgggctgg ggcacctttg tgcagcttcg 1980
   aggacaggaa aggccccaat cttgcccact ggccgctgag cgcccccgag ccctgactcc 2040
   tggactccgg aggactgagc ccccaccgga actgggctgg cgcttggatc tggggtggga 2100
   gtaacagggc agaaatgatt aaaatgtttg a 2131

<210> 62
<211> 1032
<212> DNA
<213> Homo sapiens

<400> 62
ctacgcggcc gagatctgca tcgccctcaa cttcctgcac gagaggggga tcatctacag 60
ggacctgaag ctggacaacg tcctcctgga tgcggacggg cacatcaagc tcacagacta 120

cggcatgtgc aaggaaggcc tgggccctgg tgacacaacg agcactttct gcggaacccc 180
gaattacatc gcccccgaaa tcctgcgggg agaggagtac gggttcagcg tggactggtg 240
ggcgctggga gtcctcatgt ttgagatgat ggccgggcgc tccccgttcg acatcatcac 300
cgacaacccg gacatgaaca cagaggacta ccttttccaa gtgatcctgg agaagcccat 360
ccggatcccc cggttcctgt ccgtcaaagc ctcccatgtt ttaaaaggat ttttaaataa 420
ggaccccaaa gagaggctcg gctgccggcc acagactgga ttttctgaca tcaagtccca 480
cgcgttcttc cgcagcatag actgggactt gctggagaag aagcaggcgc tccctccatt 540
ccagccacag atcacagacg actacggtct ggacaacttt gacacacagt tcaccagcga 600
gcccgtgcag ctgaccccag acgatgagga tgccataaag aggatcgacc agtcagagtt 660
cgaaggcttt gagtatatca acccattatt gctgtccacc gaggagtcgg tgtgaggccg 720
cgtgcgtctc tgtcgtggac acgcgtgatt gaccctttaa ctgtatcctt aaccaccgca 780
tatgcatgcc aggctgggca cggctccgag ggcggccagg gacagacgct tgcgccgaga 840
ccgcagaggg aagcgtcagc gggcgctgct gggagcagaa cagtccctca cacctgggcc 900
cgggcaggcc agcttcgtgc tggaggaact tgctgctgtg cctgcgtcgc ggcggatccg 960
cggggaccct gccgaggggg ctgtcatgcg gtttccaagg tgcacatttt ccacggaaac 1020
agaactcgat gc 1032

<210> 63
<211> 615
<212> DNA
<213> Homo sapiens

<400> 63
gtttctggat actgccttca atctccaggc ctttgagggc aagacatttt agagctgggc 60
aaaacctgtg taggtctcgc tgtgggtttg ctggggacca agggggtgat gaaaagggga 120
ggggcggagc tcctgcccaa gagaggggct gtggggcccc aggataaaac agacacagtg 180
acagggccaa gagccagcac tgctggcctt ggtgtcatgc cagaatctac caggactgag 240
ggagccagag gagtcctgta ggcaggctac tgtgctggag catcccccag ctgctcccat 300
cttgctggaa tttcttgggc ggcttctcca cctgtatctc aagacagaca cccgggggcc 360
tgtgtctgtg gccgctccca tcccggcagc cctggctgct gctcgcccca ccctcgctta 420
tctgtagatt caaagcgatg ttctcttctg tgctcttaga agtagggagt tcagcagtaa 480
cagccaggtg aagcgaacct gctgggtgat ttgtttgcgc tctgttttat ggggcattcc 540
tgcgagatgt gtcagcttct gtatgagatg cagccacagc tcatgtgtac caaagtagaa 600
aaccaaatca cagag 615

<210> 64
<211> 123
<212> DNA
<213> Artificial Sequence

<220>
<223> Description of Artificial Sequence:primer

<400> 64
aggcggcaac gccgaggaga ggagaggaag gccccagaaa accaggagga agaggaggag 60
cgtgcagaac tgaatcagtc ggaggaacct gaggcaggcg agagtagtac tggagggcct 120
tga 123

<210> 65
<211> 29
<212> DNA
<213> Artificial Sequence

<220>
<223> Description of Artificial Sequence:primer

<400> 65
acgtgaattc aggcggcaac gccgaggag 29

<210> 66
<211> 32
<212> DNA
<213> Artificial Sequence

<220>
<223> Description of Artificial Sequence:primer

<400> 66

cgatctcgag tcaaggccct ccagtactac tc                                    32

<210> 67
<211> 72
<212> PRT
<213> Homo sapiens

<400> 67
Lys Cys Glu Arg Val Arg Ser Trp Val Asn Phe Asp Ser Ser Arg Leu
1               5                   10                  15

Pro Gly Ser Leu Arg Ala Ser Ser Ser Ser Arg Ser Pro Val Ala Ala
            20                  25                  30

Gly Ser Val Arg Arg Ser Ala Pro Ala Leu Ala Leu Pro Trp Ala Leu
        35                  40                  45

Pro Ala Pro Pro Arg Trp Pro Ala Ser Ala Ser Gln Pro Gly Pro Gly
    50                  55                  60

Pro Gly Gly Ser Gly Ser Pro Arg
65                  70

<210> 68
<211> 13
<212> PRT
<213> Homo sapiens

<400> 68
Ser Thr Met Leu Ala Gly Leu Ala Ala Leu Phe Leu Ala
1               5                   10

<210> 69

<211> 25
<212> PRT
<213> Homo sapiens

<400> 69
Val Asn Tyr Ala Gly Trp Thr Gly Cys Leu Val Pro Gly Leu Gly Leu
1               5                   10                  15

Ser Phe Ile Thr Ile Thr Cys Thr Asp
            20                  25

<210> 70
<211> 35
<212> PRT
<213> Homo sapiens

<400> 70
Lys Asp Ser Leu Phe Asn Lys Trp Cys Trp Glu Asn Trp Ile Ser Ile
1               5                   10                  15

Cys Lys Arg Met Lys Leu Asp Leu Tyr Leu Thr Pro Leu Ala Lys Ile
            20                  25                  30

Ser Ser Lys
        35

<210> 71
<211> 11
<212> PRT
<213> Homo sapiens

<400> 71
Gln Pro Arg Pro Pro Ile Tyr Ser Ser His Leu
1               5                   10

<210> 72
<211> 49
<212> PRT
<213> Homo sapiens

<400> 72
Ile Asn Met Leu Ala Phe Ile Pro Val Leu Thr Lys Lys Ile Asn Pro
1               5                   10                  15

Arg Ser Thr Glu Ala Ala Ile Lys Tyr Phe Leu Thr Gln Ala Thr Ala
            20                  25                  30

Ser Ile Ile Leu Leu Ile Ala Ile Leu Phe Asn Asn Ile Leu Ser Gly
        35                  40                  45

Gln

<210> 73
<211> 25
<212> PRT
<213> Homo sapiens

<400> 73
Gly Glu Ala Tyr Ile Thr Asp His Phe Ser Thr Gln Lys Pro Glu Thr
1               5                   10                  15

Ser Ala Leu Ser Ser Cys Leu Gln Leu
            20                  25

<210> 74
<211> 13

<212> PRT
<213> Homo sapiens

<400> 74
Arg Gly Leu Tyr Tyr Gly Ser Phe Leu Tyr Ser Glu Thr
1               5                   10

<210> 75
<211> 37
<212> PRT
<213> Homo sapiens

<400> 75
Ser Gln Gln Ala Asn Leu Glu Tyr Pro Trp Ser Thr Pro Cys Pro Pro
1               5                   10                  15

Gly Thr Cys Pro Cys Gly Trp Arg Val Ser Arg Arg Lys Thr Leu Ala
            20                  25                  30

Pro Thr Ala Ala Pro
        35

<210> 76
<211> 59
<212> PRT
<213> Homo sapiens

<400> 76
Thr Pro Thr Pro Ser Leu Ser Ser Gln Ala Leu Pro Ser Pro Arg Leu
1               5                   10                  15

Pro Thr Thr Asp Gly Ala His Pro Gln Pro Ile Ser Pro Ile Pro Gly
            20                  25                  30

Gly Val Ser Ser Ser Gly Leu Ser Arg Met Gly Ala Val Pro Val Met
        35                  40                  45

Met Pro Ala Gln Ser Gln Ala Gly Ser Leu Val
    50                  55

<210> 77
<211> 19
<212> PRT
<213> Homo sapiens

<400> 77
Lys Tyr Lys Asn Leu Pro Gly Met Val Ala His Ala His Asn Pro Ser
1               5                   10                  15

Tyr Trp Gly

<210> 78
<211> 109
<212> PRT
<213> Homo sapiens

<400> 78
Ala Cys Ser Arg Trp Cys Ser Trp Ser Ser Pro Pro Cys Val Pro Gly
1               5                   10                  15

Ile Arg Gly Thr Cys Ser Gln Ser Ser Phe Gln Met His Pro Cys Pro
            20                  25                  30

Val Leu Pro Ile Ala Ser Ala Ala Ser Gly Arg Gly Leu Ser Ser Lys
        35                  40                  45

Val Ser Ala Val Leu Gly Ala Asp Gly Cys Leu His Gly Leu Cys Ala

    50                  55                  60

Glu Gly Gly Val Val Val Ser Val Pro Ala Gly Gly Trp Gly Ala Leu
65                  70                  75                  80

Thr Ala Leu Tyr Gln Glu Gly Gly Gly Glu Lys His Phe Ala Val Thr
                85                  90                  95

His Leu Gly Pro Thr Pro Phe Gln Gly Thr Cys Arg Cys
            100                 105

<210> 79
<211> 35
<212> PRT
<213> Homo sapiens

<400> 79
Pro Ala Gln Gly Gly Val Arg Gly Leu Arg Leu Leu Val Cys Leu Val
1               5                   10                  15

Phe Gly Val Pro Ala Arg Arg Ala His Ser Arg Cys Thr Pro Val Gln
            20                  25                  30

Cys Cys Leu
        35

<210> 80
<211> 60
<212> PRT
<213> Homo sapiens

<400> 80
Lys Val Lys Leu Leu Arg Ser Leu Pro Gln Arg Phe Lys Met Asp Val
1               5                   10                  15

His Ile Thr Pro Gly Thr His Ala Ser Glu His Ala Val Asn Lys Gln
            20                  25                  30

Leu Ala Asp Lys Glu Arg Val Ala Ala Ala Leu Glu Asn Thr His Leu
        35                  40                  45

Leu Glu Val Val Asn Gln Cys Leu Ser Ala Arg Ser
    50                  55                  60

<210> 81
<211> 35
<212> PRT
<213> Homo sapiens

<400> 81
Gln Asn Ile His Leu Ile Arg Ala Pro Leu Ala Gly Lys Gly Lys Gln
 1               5                  10                  15

Leu Glu Glu Lys Met Val Gln Gln Leu Gln Glu Asp Val Asp Met Glu
            20                  25                  30

Asp Ala Pro
        35

<210> 82
<211> 118
<212> PRT
<213> Homo sapiens

<400> 82
Phe Lys Pro Lys Pro Pro Lys Asn Glu Ser Leu Glu Thr Tyr Pro Val
 1               5                  10                  15

Met Lys Tyr Asn Pro Asn Val Leu Pro Val Gln Cys Thr Gly Lys Arg
            20                  25                  30

Asp Glu Asp Lys Asp Lys Val Gly Asn Val Glu Tyr Phe Gly Leu Gly
        35                  40                  45

Asn Ser Pro Gly Phe Pro Leu Gln Tyr Tyr Pro Tyr Tyr Gly Lys Leu
    50                  55                  60

Leu Gln Pro Lys Tyr Leu Gln Pro Leu Leu Ala Val Gln Phe Thr Asn
65                  70                  75                  80

Leu Thr Met Asp Thr Glu Ile Arg Ile Glu Cys Lys Ala Tyr Gly Glu
                85                  90                  95

Asn Ile Gly Tyr Ser Glu Lys Asp Arg Phe Gln Gly Arg Phe Asp Val
            100                 105                 110

Lys Ile Glu Val Lys Ser
        115

<210> 83
<211> 98
<212> PRT
<213> Homo sapiens

<400> 83
Asn Glu Ser Glu Val Lys Gly Tyr Lys Val Leu Tyr Arg Trp Asn Arg
1               5                   10                  15

Gln Ser Ser Thr Ser Val Ile Glu Thr Asn Lys Thr Ser Val Glu Leu
            20                  25                  30

Ser Leu Pro Phe Asp Glu Asp Tyr Ile Ile Glu Ile Lys Pro Phe Ser
        35                  40                  45

Asp Gly Gly Asp Gly Ser Ser Ser Glu Gln Ile Arg Ile Pro Lys Ile
    50                  55                  60

Ser Asn Ala Tyr Ala Arg Gly Ser Gly Ala Ser Thr Ser Asn Ala Cys
65                  70                  75                  80

Thr Leu Ser Ala Ile Ser Thr Ile Met Ile Ser Leu Thr Ala Arg Ser
                85                  90                  95

Ser Leu

<210> 84
<211> 147
<212> PRT
<213> Homo sapiens

<400> 84
Gly Ile Gln Ser Thr Pro Leu Asn Leu Ala Val Asn Trp Arg Cys Glu
1               5                   10                  15

Pro Ser Ser Thr Asp Leu Arg Ile Asp Tyr Lys Tyr Asn Thr Asp Ala
            20                  25                  30

Met Thr Thr Ala Val Ala Leu Asn Asn Val Gln Phe Leu Val Pro Ile
        35                  40                  45

Asp Gly Gly Val Thr Lys Leu Gln Ala Val Leu Pro Pro Ala Val Trp
    50                  55                  60

Asn Ala Glu Gln Gln Arg Ile Leu Trp Lys Ile Pro Asp Ile Ser Gln
65                  70                  75                  80

Lys Ser Glu Asn Gly Gly Val Gly Ser Leu Leu Ala Arg Phe Gln Leu

                85                  90                  95

Ser Glu Gly Pro Ser Lys Pro Ser Pro Leu Val Val Gln Phe Thr Ser
            100                 105                 110

Glu Gly Ser Thr Leu Ser Gly Cys Asp Ile Glu Leu Val Gly Ala Gly
        115                 120                 125

Tyr Arg Phe Ser Leu Ile Lys Lys Arg Phe Ala Ala Gly Lys Tyr Leu
    130                 135                 140

Ala Asp Asn
145

<210> 85
<211> 76
<212> PRT
<213> Homo sapiens

<400> 85
Met Ser Lys Ala His Pro Pro Glu Leu Lys Lys Phe Met Asp Lys Lys
1               5                   10                  15

Leu Ser Leu Lys Leu Asn Gly Gly Arg His Val Gln Gly Ile Leu Arg
            20                  25                  30

Gly Phe Asp Pro Phe Met Asn Leu Val Ile Asp Glu Cys Val Glu Met
        35                  40                  45

Ala Thr Ser Gly Gln Gln Asn Asn Ile Gly Met Val Val Ile Arg Gly
    50                  55                  60

Asn Ser Ile Ile Met Leu Glu Ala Leu Glu Arg Val
65                  70                  75

<210> 86
<211> 240
<212> PRT
<213> Homo sapiens

<400> 86
Pro Asp Pro Arg Pro Ser Pro Pro Arg Pro Asp Val Cys Met Ala Asp
1               5                   10                  15

Pro Glu Gly Leu Ser Ser Glu Ser Gly Arg Val Glu Arg Leu Arg Glu
            20                  25                  30

Lys Glu Lys Val Gln Gly Arg Val Gly Arg Arg Ala Pro Gly Lys Ala
        35                  40                  45

Lys Pro Ala Ser Pro Ala Arg Arg Leu Asp Leu Arg Gly Lys Arg Ser
    50                  55                  60

Pro Thr Pro Gly Lys Gly Pro Ala Asp Arg Ala Ser Arg Ala Pro Pro
65                  70                  75                  80

Arg Pro Arg Ser Thr Thr Ser Gln Val Thr Pro Ala Glu Glu Lys Asp
                85                  90                  95

Gly His Ser Pro Met Ser Lys Gly Leu Val Asn Gly Leu Lys Ala Gly
            100                 105                 110

Pro Met Ala Leu Ser Ser Lys Gly Ser Ser Gly Ala Pro Val Tyr Val
        115                 120                 125

Asp Leu Ala Tyr Ile Pro Asn His Cys Ser Gly Lys Thr Ala Asp Leu
    130                 135                 140

Asp Phe Phe Arg Arg Val Arg Ala Ser Tyr Tyr Val Val Ser Gly Asn
145                 150                 155                 160

Asp Pro Ala Asn Gly Glu Pro Ser Arg Ala Val Leu Asp Ala Leu Leu
                165                 170                 175

Glu Gly Lys Ala Gln Trp Gly Glu Asn Leu Gln Val Lys Val Thr Leu
            180                 185                 190

Ile Pro Thr His Asp Thr Glu Val Thr Arg Glu Trp Tyr Gln Gln Thr
        195                 200                 205

His Glu Gln Gln Gln Gln Leu Asn Val Leu Val Leu Ala Ser Ser Ser
    210                 215                 220

Thr Val Val Met Gln Asp Glu Ser Phe Pro Ala Cys Lys Ile Glu Phe
225                 230                 235                 240

<210> 87
<211> 183
<212> DNA
<213> Homo sapiens

<400> 87
aaggtcaagc ttctgcgctc ccttcctcag cgtttcaaga tggacgtgca cattactccg 60
gggacccatg cctcagagca tgcagtgaac aagcaacttg cagataagga gcgggtggca 120
gctgccctgg agaacaccca cctcttggag gttgtgaatc agtgcctgtc agcccgctcc 180
tga                                                               183

<210> 88
<211> 108
<212> DNA
<213> Homo sapiens

<400> 88
caaaacatcc atcttatccg agcccctctt gcaggcaaag ggaaacagtt ggaagagaaa 60
atggtacagc agttacaaga ggatgtggac atggaagatg ctccttaa 108

<210> 89
<211> 357
<212> DNA
<213> Homo sapiens

<400> 89
ttcaaaccta agcctcccaa gaatgagtcc ttggagactt acccagtgat gaagtataac 60
ccaaatgtcc ttcccgttca gtgcactggc aagcgagatg aagataagga taaagttgga 120
aatgtggagt attttggact gggcaactcc cctggttttc ctctgcagta ttatccgtac 180
tatggcaaac tcctgcagcc caaatacctg cagcccctgc tggccgtaca gttcaccaat 240
cttaccatgg acactgaaat tcgcatagag tgtaaggcgt acggtgagaa cattgggtac 300
agtgagaaag accgttttca gggacgtttt gatgtaaaaa ttgaagttaa gagctga 357

<210> 90
<211> 297
<212> DNA
<213> Homo sapiens

<400> 90
aatgagtcgg aagtaaaagg atacaaagtc ttgtacagat ggaacagaca aagcagcaca 60
tctgtcattg aaacaaataa aacatcggtg gagctttctt tgcctttcga tgaagattat 120
ataatagaaa ttaagccatt cagcgacgga ggagatggca gcagcagtga acaaattcga 180
attccaaaga tatcaaatgc ctacgcgaga ggatctgggg cttccacttc gaatgcatgt 240
acgctgtcag ccatcagtac aataatgatt tccctcacag ctaggtccag tttatga 297

<210> 91
<211> 444
<212> DNA

<213> Homo sapiens

<400> 91
ggcattcagt ccacacctct gaacctggca gtgaattggc gatgtgagcc ttcaagcact 60
gacctgcgca tagattacaa atataataca gatgcaatga cgactgctgt ggccctcaac 120
aatgtgcagt tcctggtccc catcgacgga ggagtcacca agctccaggc agtgctccca 180
ccagcagtct ggaatgctga acaacagaga atattgtgga agattcctga tatctctcag 240
aagtcagaaa atggaggggt gggttctttg ttggcaagat ttcagttatc tgaaggccca 300
agcaaacctt ctccattggt tgtgcagttc acaagtgaag gaagcaccct ttctggctgt 360
gacattgaac ttgttggagc agggtatcga ttttcactca tcaagaaaag gtttgctgca 420
ggaaaatact tggcagataa ctaa 444

<210> 92
<211> 231
<212> DNA
<213> Homo sapiens

<400> 92
atgagcaaag ctcaccctcc cgagttgaaa aaatttatgg acaagaagtt atcattgaaa 60
ttaaatggtg gcagacatgt ccaaggaata ttgcggggat ttgatccctt tatgaacctt 120
gtgatagatg aatgtgtgga gatggcgact agtggacaac agaacaatat tggaatggtg 180
gtaatacgag gaaatagtat catcatgtta gaagccttgg aacgagtata a 231

<210> 93
<211> 723
<212> DNA
<213> Homo sapiens

<400> 93
ccagaccccc gcccatcccc tccccgccct gatgtgtgca tggctgaccc cgaggggctc 60
agctcagagt ctgggagagt agagaggcta cgggagaagg aaaaggttca ggggcgagta 120
gggcgcaggg ccccaggcaa ggccaagcca gcgtcccctg cacggcgtct ggatcttcgg 180
ggaaaacgct cacccacccc tggtaaaggg cctgcagatc gagcatcccg ggccccacct 240
cgaccacgca gcaccacaag ccaggtcacc ccagcagagg aaaaggatgg acacagcccc 300

atgtccaaag gcctagtcaa tggactcaag gcaggaccaa tggccttgag ttccaagggc 360
agctctggtg cccctgtata tgtggatctc gcctacatcc cgaatcattg cagtggcaag 420
actgctgacc ttgacttctt ccgtcgagtg cgtgcatcct actatgtggt cagtgggaat 480
gaccctgcca atggcgagcc aagccgggct gtgctggatg ccctgctgga gggcaaggcc 540
cagtgggggg agaatcttca ggtgaaggtg actctgatcc ctactcatga cacggaggtg 600
actcgtgagt ggtaccaaca aactcatgag cagcagcaac aactgaatgt cctggtcctg 660
gctagcagca gcaccgtggt gatgcaggat gagtccttcc ctgcctgcaa gattgagttc 720
tga 723